# EUROPÄISCHE PATENTSCHRIFT

(11) **EP 3 936 030 B1**
(45) Veröffentlichungstag und Bekanntmachung des Hinweises auf die Patenterteilung: **01.10.2025**
(21) Anmeldenummer: 20184580.7
(22) Anmeldetag: 07.07.2020
(51) Int. Cl.: A61B 5/00, A61B 5/021, A41D 13/12

(54) **NICHT-INVASIVES BLUTDRUCKMESSGERÄT**
NON-INVASIVE BLOOD PRESSURE MONITOR
TENSIOMÈTRE NON INVASIF

(43) Veröffentlichungstag der Anmeldung: 12.01.2022
(73) Patentinhaber: von Lilienfeld-Toal, Sophie, 37124 Rosdorf (DE)
(72) Erfinder: v. Lilienfeld-Toal, Hermann, 63571 Gelnhausen (DE); Kopatsch, Jens, Shanghai, 200009 (CN); Baulmann, Johannes, 53113 Bonn (DE)
(74) Vertreter: Vossius & Partner Patentanwälte Rechtsanwälte mbB

(56) Entgegenhaltungen:
- EP-A1- 2 591 723
- EP-A2- 2 324 761
- CN-A- 108 618 772
- CN-A- 109 381 170
- US-A1- 2006 253 041
- US-A1- 2007 215 836
- US-A1- 2014 296 749
- US-A1- 2014 331 412
- US-A1- 2016 018 274
- US-A1- 2017 079 868
- US-A1- 2019 069 785
- US-A1- 2020 121 201

## Beschreibung

Nicht-invasive Blutdruckmessgeräte, sogenannte indirekte Blutdruckmessgeräte, werden in der medizinischen Versorgung eingesetzt, um den Blutdruck, insbesondere den arteriellen Blutdruck, eines Patienten zu bestimmen.

US 2007/215836 A1 offenbart ein Kleidungsstück aus Filamenten oder Fasern zum Bilden eines textilen Blutdruckmessgeräts zur Messung eines Blutdrucks. EP 2 324 761 A2 offenbart ein Kleidungsstück mit eingebetteten Sensoren zum Aufzeichnen eines Blutdrucks. EP 2 591 723 A1 offenbart ein textiles Produkt mit einem darin eingearbeiteten Drucksensor. Der Drucksensor umfasst einen mehrlagigen Faden mit einer druckempfindlichen Schicht, die einen druckabhängigen elektrischen Widerstand aufweist, und einer leitfähigen Schicht, die mit der druckempfindlichen Schicht in Kontakt steht. US 2006/253041 A1 offenbart ein Blutdruckmessgerät mit einer viskoelastischen Manschette, die einen Körperteil aufgrund ihrer Viskoelastizität elastisch zusammendrückt und dekomprimiert, einem Blutdrucksensor zum Messen von Blutdruckschwankungen und einem Blutflusssensor zum Messen von Blutflussschwankungen. CN 108 618 772 A offenbart ein System zur kontinuierlichen Blutdrucküberwachung. Das System umfasst ein Blutdrucküberwachungsgerät und eine Trageeinheit. Die Trageeinheit umfasst einen festen Haltering, der innen mit einem Hohlraum versehen ist, eine Einstellvorrichtung, einen beweglichen Haltering und eine Luftbefüllungsvorrichtung.

Insbesondere soll dadurch chronischer Bluthochdruck, sogenannte arterielle Hypertonie, oder chronischer Blutniederdruck, sogenannte arterielle Hypotonie, festgestellt werden können.

Bluthochdruck und Blutniederdruck können auf Dauer die Blutgefäße schädigen und tragen häufig zur Entstehung von Folgeerkrankungen bei, wie beispielsweise ein Herzinfarkt oder ein Schlaganfall.

Deshalb ist das frühzeitige Feststellen eines Bluthochdrucks und eines Blutniederdrucks eine wichtige diagnostische Maßnahme, um Folgeerkrankungen durch medizinische Behandlung vorbeugen zu können.

Nicht-invasive Blutdruckmessgeräte umfassen in der Regel eine Messmanschette, die um den Arm eines Patienten angelegt wird. Um den Blutdruck messen zu können, wird mittels der angelegten Messmanschette ein Druck auf die Oberarmarterie des Patienten ausgeübt, der oberhalb des arteriellen Blutdrucks liegt, sodass dadurch der Blutfluss in der Oberarmarterie unterbunden werden kann.

Anschließend wird der Druck der Messmanschette auf den Arm des Patienten allmählich reduziert, bis ein wiederhergestellter Blutfluss durch die Oberarmarterie detektiert wird. Dieser Zustand wird in der Regel durch das Detektieren von Blutströmungsgeräuschen festgestellt (auskultatorische Messung). Der Druck, der beim Erreichen dieses Zustands in der Messmanschette herrscht, entspricht dem Blutdruck des Patienten, wobei zwischen systolischem und diastolischem Druck unterschieden wird.

Der Druckaufbau in der Messmanschette wird in der Regel durch Befüllen einer in der Messmanschette angeordneten Druckluftkammer mit Luft bewirkt. Die Druckluftkammer kann zum einen manuell mittels einer Handpumpe mit Luft befüllt werden. Alternativ kann ein Druckluftkompressor zum Befüllen der Druckluftkammer mit Luft eingesetzt werden.

Derartige Blutdruckmessungen werden in der Regel in größeren Zeitabständen durchgeführt, beispielsweise bei jährlichen oder halbjährlichen ärztlichen Vorsorgeuntersuchungen.

Der Blutdruck eines Menschen kann jedoch durch körperliche Anstrengung, Stress, Schmerzen, starke Hitze oder Kälte beeinflusst werden und kann sich demzufolge situationsbedingt ändern. Deshalb kann der Blutdruck als ein genereller Indikator für den allgemeinen Gesundheitszustand des Menschen dienen.

Die vorstehend beschriebenen nicht-invasiven Blutdruckmessgeräte werden deshalb auch zur Langzeitüberwachung des Blutdrucks eingesetzt, um den Blutdruck eines Patienten über einen längeren Zeitraum zu kontrollieren.

Dabei führt der Patient ein mobiles Blutdruckmessgerät mit sich, welches den Blutdruck in vorbestimmten zeitlichen Abständen, in der Regel in Abständen von 15 oder 30 Minuten, bestimmt.

Diese Art von Langzeitblutdruckmessung weist jedoch einen geringen Tragekomfort für den Patienten auf. Zum einen muss ein Druckluftkompressor zum Befüllen der Kammer der Messmanschette von dem Patienten mitgeführt werden. Des Weiteren wird der Komfort des Patienten durch den Lärm des Druckluftkompressors beim Befüllen der Kammer der Messmanschette und durch die wechselnden Druckzustände am Arm des Patienten beeinträchtigt.

Eine derartige Langzeitblutdruckmessung, die in der Regel auch im schlafenden Zustand des Patienten durchgeführt wird, führt deshalb häufig zu Schlafstörungen, wodurch das allgemeine Wohlbefinden des Patienten beeinträchtigt wird.

Außerdem wird durch eine solche Langzeitüberwachung des Blutdrucks mittels des oben beschriebenen Blutdruckmessgeräts nur eine periodische Überwachung des Blutdrucks in vordefinierten zeitlichen Abständen ermöglicht. Eine kontinuierliche Blutdruckmessung ist wegen des durch den Druckaufbau der Messmanschette bewirkten Blutstaus in den Blutgefäßen nicht möglich.

Deshalb können mittels Langzeitblutdruckmessungen mit den derzeit verfügbaren Blutdruckmessgeräten die Auswirkungen von situationsbedingten Faktoren, wie beispielsweise körperliche Anstrengung oder Stress, auf den Blutdruck nicht oder nicht vollständig erfasst werden.

Des Weiteren ist der Patient in der Regel bei der Anwendung von konventionellen Blutdruckmessgeräten und bei der Auswertung der mittels des Blutdruckmessgeräts gemessenen Daten auf medizinisches Personal angewiesen. Selbstvermessungen des Blutdrucks durch den Patienten selbst sind somit nicht oder zumindest nur eingeschränkt möglich.

Deshalb ist es Aufgabe der vorliegenden Erfindung, die Nachteile des Standes der Technik zu überwinden oder zumindest eine Alternative bereitzustellen und insbesondere, die Überwachung des Blutdrucks zu verbessern.

Diese Aufgabe wird durch die Gegenstände der unabhängigen Patentansprüche gelöst. Vorteilhafte Ausgestaltungen mit zweckmäßigen Weiterbildungen der Erfindung sind in den jeweiligen Unteransprüchen sowie in der nachfolgenden Beschreibung angegeben.

Die Erfindung betrifft ein Blutdruckmessgerät zum nicht-invasiven Blutdruckmessen. Das Blutdruckmessgerät umfasst eine Messmanschette, die dazu eingerichtet ist, wenigstens bereichsweise um einen Umfang einer Extremität eines Patienten angelegt zu werden. Die Messmanschette weist wenigstens eine proximale Schicht und eine distale Schicht auf.

Unter proximal ist im Sinne der vorliegenden Erfindung eine zur Extremität des Patienten hin gerichtete Anordnung zu verstehen. Unter distal, hingegen, ist eine von der Extremität des Patienten weggerichtete Anordnung zu verstehen. Entsprechend ist die proximale Schicht der an der Extremität des Patienten angelegten Messmanschette näher an der Extremität des Patienten angeordnet als die distale Schicht.

Vorzugsweise umgreift die distale Schicht die proximale Schicht vollständig, in Umfangs- und/oder Längenrichtung. Entsprechend umfasst die distale Schicht vorzugsweise eine Gesamtfläche, die mindestens genauso groß ist, wie die Gesamtfläche der proximalen Schicht. Vorzugsweise umfasst die distale Schicht eine Gesamtfläche auf, die größer ist als die Gesamtfläche der proximalen Schicht.

Die proximale Schicht kann ein druckempfindliches, vorzugsweise zumindest teilweise komprimierbares, Element aufweisen, das dazu eingerichtet ist, unter Druckeinfluss ein diesem Druckeinfluss äquivalentes, kontinuierliches Drucksignal zu erzeugen.

Mittels des druckempfindlichen Elements der proximalen Schicht ist es möglich, die Intensität von Druckpulsen in der Extremität des Patienten, die periodisch durch den arteriellen Blutdruck des Patienten hervorgerufen werden, zu erfassen. Diese erfassten Druckänderungen generieren ein kontinuierliches Drucksignal, welches zu dem Druckeinfluss äquivalent ist.

Die Erfassung der Intensität von Druckpulsen in der Extremität des Patienten kann vorzugsweise dadurch erfolgen, dass zumindest ein Bereich des druckempfindlichen Elements komprimiert bzw. ausgelenkt wird. Das druckempfindliche Element kann beispielsweise ein piezoelektrisches Material umfassen, das unter Druckeinwirkung komprimiert werden und dabei eine messbare Ladungsänderung des piezoelektrischen Materials hervorruft, um daraus ein Drucksignal generieren zu können. Alternativ oder zusätzlich kann das druckempfindliche Element ein oder mehrere Dehnungsmessstreifen und/oder ein oder mehrere druckempfindliche Halbleiterchips und/oder ein oder mehrere Hall-Elemente aufweisen.

Ferner kann das druckempfindliche Element ein durch eine Umhüllung, beispielsweise in Form einer Polymerbeschichtung, eingehüllter elektrischer Leiter sein. Vorzugsweise können in diesem Fall mindestens zwei elektrische Leiter vorgesehen sein, wobei zumindest einer der elektrischen Leiter eine Umhüllung aufweist, wobei die Umhüllung vorzugsweise zumindest eine geringe elektrische Leitfähigkeit aufweist. Dabei können die elektrischen Leiter in der proximalen Schicht mit zumindest geringem Abstand zueinander angeordnet sein. Vorzugsweise können sich die elektrischen Leiter zumindest bereichsweise kreuzen oder zumindest überlagern.

Durch eine Druckeinwirkung wird der Abstand der elektrischen Leiter zueinander reduziert, vorzugsweise durch Kompression der Umhüllung des zumindest einen elektrisch leitfähigen Leiters. Dadurch sinkt der vor allem durch die Umhüllung bewirkte elektrische Widerstand zwischen den elektrischen Leitern. Diese Reduktion des elektrischen Widerstands kann gemessen werden. Daraus kann wiederum ein Drucksignal generiert werden.

Der elektrische Leiter kann vorzugsweise ein elektrisch leitfähiger Faden, ein elektrisch leitfähiges Band oder eine elektrisch leitfähige Folie sein. Alternativ oder zusätzlich kann der elektrische Leiter selbst komprimierbar bzw. auslenkbar sein.

Die distale Schicht kann zumindest in Umfangsrichtung unter Druckeinfluss im Wesentlichen nicht dehnbar sein. Mit anderen Worten ist die distale Schicht jedenfalls in tangentialer Richtung und damit entlang der Umfangsrichtung im Wesentlichen nicht dehnbar. Vorzugsweise ist die distale Schicht auch in Richtung ihrer Längserstreckung, also in Längenrichtung der Extremität, im Wesentlichen nicht dehnbar. Die fehlende Dehnbarkeit, insbesondere in Umfangsrichtung, erlaubt es der distalen Schicht einen Widerstand bzw. Anschlag zu bilden, gegen den die proximale Schicht gedrückt wird. So kann die proximale Schicht Druckänderungen in der bzw. durch die Extremität detektieren, wie auch nachfolgend beschrieben. Außerdem kann das Blutdruckmessgerät eine Auswertevorrichtung umfassen, die dazu eingerichtet ist, in Abhängigkeit von dem Drucksignal den Blutdruck des Patienten zu bestimmen.

Die in Umfangsrichtung unter Druckeinfluss im Wesentlichen nicht dehnbare distale Schicht dient dazu, eine radiale Auslenkung der proximalen Schicht durch den Druckeinfluss auf die proximale Schicht im angelegten Zustand zu verhindern. Dadurch können Druckeinflüsse auf die proximale Schicht vollständig oder zumindest nahezu vollständig erfasst werden.

Durch die Verwendung des druckempfindlichen Elements zum Detektieren des arteriellen Blutdrucks kann das vorliegende Blutdruckmessgerät im Vergleich zu den eingangs beschriebenen konventionellen Blutdruckmessgeräten einen geringeren Anlegedruck aufweisen, der beispielweise geringer ist als der arterielle Blutdruck, da im Gegensatz zum konventionellen Messprinzip kein arterieller Blutstau bewirkt werden muss.

Deshalb kann der Anlegedruck wesentlich geringer eingestellt werden als bei dem konventionellen Blutdruckmessprinzip. Außerdem kann der Anlegedruck der Messmanschette auf einen konstanten Wert während der gesamten Tragedauer eingestellt werden. Bei konventionellen Blutdruckmessgeräten wird der Anlegedruck periodisch durch das Befüllen der Druckluftkammer erhöht und anschließend wieder reduziert. Dies führt zu einer erheblichen Beeinträchtigung des Patienten und zu einer Aufwändigen Apparatur, die der Patient mit sich führen muss.

Außerdem ermöglicht das vorliegende Blutdruckmessgerät im Gegensatz zu den eingangs beschriebenen konventionellen Blutdruckmessgeräten eine kontinuierliche Messung des Blutdrucks. Somit kann der Blutdruck kontinuierlich überwacht werden und als verlässlicher Indikator, auch für das situationsabhängige Wohlbefinden des Patienten dienen.

Dadurch ist das Blutdruckmessgerät auch vielfältig einsetzbar. Beispielsweise kann das Blutdruckmessgerät bei Freizeittätigkeiten, beispielsweise beim Sport, getragen werden, um den Blutdruck während der Tätigkeit zu überwachen. Denkbar ist jedoch auch die Anwendung des Blutdruckmessgeräts bei gewerblichen Tätigkeiten. Beispielsweise kann das Blutdruckmessgerät von Mitarbeitern eines Betriebs getragen werden, um somit beispielsweise den Blutdruck bei körperlich anstrengenden Tätigkeiten, beispielsweise bei der Bedienung einer Maschinenanlage, zu überwachen. Denkbar ist jedoch auch ein militärischer Einsatz des Blutdruckmessgeräts, um beispielsweise bei militärischen Übungen die körperliche Verfassung von Militärangehörigen anhand von gemessenen Blutdruckwerten zu bewerten.

Das Blutdruckmessgerät kann außerdem für eine engmaschige Überwachung von Patienten mit kritischen medizinischen Zuständen verwendet werden, beispielsweise zur Betreuung eines Patienten nach einem Schlaganfall und/oder einem Herzinfarkt oder zur Betreuung eines Patienten mit einem erhöhten Risiko für einen potenziellen Schlaganfall und/oder Herzinfarkt.

Des Weiteren ist die Blutdruckmessung mittels des hierin offenbarten Blutdruckmessgeräts im Vergleich zu konventionellen Blutdruckmessgeräten benutzerfreundlicher, sodass der Patient Selbstvermessungen des Blutdrucks durchführen kann, ohne dass der Patient auf medizinisches Personal im Hinblick auf die Durchführung der Messung und die Auswertung der gemessenen Daten angewiesen ist.

Im Hinblick auf die distale Schicht ist unter im Wesentlichen nicht dehnbar im Sinne der vorliegenden Offenbarung eine Eigenschaft der distalen Schicht zu verstehen, wonach der Druckeinfluss, der im angelegten Zustand der Messmanschette von dem arteriellen Blutdruck in der Extremität des Patient radial zur Umfangsrichtung der Messmanschette auf die distale Schicht wirkt, keine oder nur eine geringe Dehnung der distalen Schicht in Umfangsrichtung hervorruft. Entsprechendes gilt vorzugsweise auch für gelegentliche Muskelkontraktionen.

Vorzugsweise kann die distale Schicht eine vordefinierte maximale relative Dehnung ε aufweisen, die als Prozentverhältnis der Änderung der Umfangserstreckung der distalen Schicht unter Druckeinfluss zur Umfangserstreckung der distalen Schicht ohne Druckeinfluss definiert ist. Vorzugsweise weist die distale Schicht eine maximale relative Dehnung ε von 7% oder weniger, bevorzugt 5% oder weniger, besonders bevorzugt 3% oder weniger und weiter bevorzugt weniger als 1%.

Vorzugsweise weist die distale Schicht ein Elastizitätsmodul E in Umfangserstreckung der distalen Schicht von mindestens 50.000 N/mm², bevorzugter mindestens 75.000 N/mm², bevorzugter mindestens 100.000 N/mm², besonders bevorzugt mindestens 125.000 N/mm², auf.

Die Auswertevorrichtung zum Bestimmen des Blutdrucks des Patienten in Abhängigkeit von dem Drucksignal kann vorteilhaft in oder an der Messmanschette angeordnet sein. Denkbar ist auch, dass die Auswertevorrichtung als integrales Bauteil der Messmanschette ausgeführt werden kann.

Alternativ kann die Auswertevorrichtung im Wesentlichen unabhängig von der Messmanschette tragbar sein, beispielsweise in einer Tasche oder an einem Gürtel des Patienten. Die Auswertevorrichtung kann hierzu über ein Verbindungskabel mit der Messmanschette verbunden sein. Denkbar ist jedoch auch eine kabellose Kommunikationsverbindung zwischen der Auswertevorrichtung und der Messmanschette, beispielsweise mittels Bluetooth, Wifi oder Near-Field-Communication (NFC).

Die Auswertevorrichtung kann ein installiertes Softwareprogramm aufweisen, die den Blutdruck des Patienten in Abhängigkeit von dem Drucksignal bestimmt und dem Patienten die bestimmten Werte anzeigen kann.

Die Applikation kann vorteilhaft dazu eingerichtet sein, von dem Patienten individuell konfiguriert zu werden. Beispielsweise kann die Applikation verschiedene auswählbare Auswertemodi und/oder Anzeigemodi aufweisen.

Alternativ kann die Auswertevorrichtung auch ein externes Gerät sein, welches nicht Teil des Blutdruckmessgeräts ist. Beispielsweise kann die Auswertevorrichtung auch ein Smartphone, ein Tablet oder ein PC des Patienten sein. Hierzu kann auf dem entsprechenden Gerät eine Applikation installierbar sein, welches den Blutdruck des Patienten in Abhängigkeit von dem Drucksignal bestimmen und dem Patienten die bestimmten Werte anzeigen kann.

Die Applikation kann vorteilhaft dazu eingerichtet sein, von dem Patienten individuell konfiguriert zu werden. Beispielsweise kann die Applikation verschiedene auswählbare Auswertemodi und/oder Anzeigemodi aufweisen.

Denkbar ist auch eine Übertragung der gemessenen Blutdruckwerte an einen zentralen Speicher, beispielsweise an einen Server oder an eine Cloud. Dort können die gemessenen Blutdruckwerte gespeichert und/oder ausgewertet werden und von dem Patienten abgerufen werden.

In einer vorteilhaften Ausgestaltung weist die Messmanschette im angelegten Zustand eine sich in Richtung der Längserstreckung der Extremität erstreckende Länge auf, die maximal 13cm, vorzugsweise maximal 10 cm, bevorzugt maximal 8 cm, besonders bevorzugt maximal 5 cm beträgt.

Konventionelle Blutdruckmessgeräte weisen in der Regel eine minimale Länge von ca. 15 cm auf, um eine Einschnürung der Extremität und die dadurch entstehende Beeinträchtigung des Patienten durch die Messmanschette zu begrenzen.

Durch die Erfassung des Blutdrucks mittels des druckempfindlichen Elements entsteht eine derartige Beeinträchtigung, beispielsweise durch Einschnürung, jedoch nicht, sodass die Länge der Messmanschette im Vergleich zu konventionellen Blutdruckmessgeräten reduziert werden kann.

Durch die Reduzierung der Länge der Messmanschette wird die Beeinträchtigung des Patienten gegenüber konventionellen Blutdruckmessgeräten reduziert, wodurch der Komfort des Patienten beim Tragen der Messmanschette, vor allem beim Langzeittragen, wesentlich erhöht wird.

In einer vorteilhaften Ausgestaltung ist das druckempfindliche Element dazu eingerichtet ist, das Drucksignal unter dem Einfluss eines Drucks, der unterhalb des systolischen Blutdrucks liegt, bevorzugt unterhalb des diastolischen Blutdrucks, zu erzeugen.

Im Vergleich zu konventionellen Druckmessgeräten, ist das hierin beschriebene Blutdruckmessgerät nicht auf die Erzeugung eines Drucks, der dem systolischen Blutdruck bzw. dem diastolischen Blutdruck entspricht und/oder diesen jeweils überschreitet, angewiesen. Bei konventionellen Blutdruckmessgeräten muss mittels der angelegten Messmanschette ein Druck auf die Oberarmarterie des Patienten ausgeübt werden, der oberhalb des arteriellen Drucks liegt, sodass der Blutfluss in der Oberarmarterie unterbunden wird, wie eingangs beschrieben.

Beim vorliegenden Blutdruckmessgerät wirkt der arterielle Blutdruck jedoch direkt auf das druckempfindliche Element, welches den arteriellen Blutdruck erfasst.

Dadurch kann der Druck, der durch die Messmanschette auf die Extremität des Patienten ausgeübt wird, reduziert werden. Dadurch wird der Tragekomfort der Messmanschette erhöht.

In einer vorteilhaften Ausgestaltung weist das druckempfindliche Element mindestens zwei elektrisch leitfähige Fäden auf, die einen Teil eines Stromkreises bilden, dessen elektrischer Widerstand durch den Druckeinfluss veränderbar ist. Diese sind Teil eines elektrischen Stromkreises, dessen elektrischer Widerstand durch den Druckeinfluss veränderbar ist.

Die elektrisch leitfähigen Fäden weisen vorzugsweise einen elektrisch leitfähigen Kern, vorzugsweise aus einem metallischen Material, auf. Zumindest einer der elektrisch leitfähigen Kerne kann vorzugsweise durch eine Umhüllung, vorzugsweise aus Polymer, eingehüllt sein. Die Umhüllung kann vorzugsweise eine zumindest geringe elektrische Leitfähigkeit aufweisen.

Dabei können die elektrisch leitfähigen Fäden in der proximalen Schicht mit geringem oder keinem Abstand zueinander angeordnet werden. Vorzugsweise können sich die elektrisch leitfähigen Fäden zumindest bereichsweise kreuzen oder zumindest überlagern.

Durch eine Druckeinwirkung kann der Abstand der elektrisch leitfähigen Fäden zumindest bereichsweise zueinander reduziert werden, vorzugsweise durch Kompression der Umhüllung des zumindest einen elektrisch leitfähigen Fadens. Dadurch sinkt der elektrische Widerstand des Stromflusses zwischen den elektrisch leitfähigen Fäden. Somit können die beiden elektrisch leitfähigen Fäden zusammen effektiv eine Stromleitung bilden, deren elektrischer Widerstand mittels der Kompression der Umhüllung veränderbar ist. Die Veränderung des elektrischen Widerstands kann gemessen werden, um daraus ein Drucksignal generieren zu können.

Denkbar ist auch die Verwendung von Carbon-Nano-Tubes in den elektrisch leitfähigen Fäden. Carbon-Nanotubes (CNT) weisen eine hohe elektrische Leitfähigkeit auf und sind deshalb für die vorliegenden Messzwecke besonders geeignet. Denkbar ist jedoch auch die Verwendung von Leitfähigkeitsruß (Carbon Black) in den elektrisch leitfähigen Fäden.

Die elektrisch leitfähigen Fäden sind vorzugsweise mit einer Spannungsquelle verbunden. Somit können die elektrisch leitfähigen Fäden zugleich ein Druckerfassungsbauteil zum Erfassen des Blutdrucks und eine Stromversorgung des Druckerfassungsbauteils bilden. Dadurch kann auf einen zusätzlichen Anschlusskabelbaum der Messmanschette verzichtet werden. Entsprechend kann das Blutdruckmessgerät kompakter und platzsparender gebaut werden. Außerdem ist dadurch das Tragen des Blutdruckmessgeräts an dem Patienten komfortabler.

Alternativ kann jedoch auch ein zusätzlicher Anschlusskabelbaum der Messmanschette vorgesehen sein.

Alternativ kann das druckempfindliche Element auch nur einen elektrisch leitfähigen Faden aufweisen. In diesem Fall kann der elektrisch leitfähige Faden eine druckempfindliche Umhüllung aufweisen, die vorzugsweise unter Druckeinfluss komprimierbar ist. Die druckempfindliche Umhüllung kann dazu eingerichtet sein, den Druckeinfluss zu detektieren und ein diesem Druckeinfluss äquivalentes, kontinuierliches Drucksignal zu erzeugen. Beispielsweise kann die druckempfindliche Umhüllung hierzu ein piezoelektrisches Material und/oder Dehnungsmessstreifen und/oder druckempfindliche Halbleiterchips aufweisen.

Vorzugsweise sind die elektrisch leitfähigen Fäden zumindest in einem Bereich der Messmanschette sich kreuzend angeordnet und bilden mindestens eine Kreuzungsstelle, an der sich mindestens zwei elektrisch leitfähige Fäden kreuzend überlagern.

Vorzugsweise bilden die elektrisch leitfähigen Fäden mehrere Kreuzungsstellen, die vorzugsweise in der proximalen Schicht verteilt angeordnet sind.

Durch die Kreuzungsstellen, die durch sich überlagernde Bereiche der Fäden gebildet werden, wird eine hohe Druckempfindlichkeit an diesen Kreuzungsstellen bewirkt, da die Umhüllung der elektrisch leitfähigen Fäden an den Kreuzungsstellen durch den Druckeinfluss des arteriellen Blutdrucks stärker komprimiert werden kann als in den Bereichen der Fäden, die sich nicht kreuzen. Dadurch nimmt der elektrische Widerstand zwischen den elektrisch leitfähigen Fäden ab. Diese Reduktion des elektrischen Widerstands kann beispielsweise mittels einer Widerstandsmessung und/oder einer Strommessung quantifiziert werden, um daraus ein Drucksignal zu generieren.

Dieses Messprinzip ist beispielsweise in der EP 2 591 723 A1 beschrieben.

Die Messempfindlichkeit kann durch die vorgesehene Anzahl an Kreuzungsstellen und/oder deren Anordnungsdichte gesteuert werden. Je höher die Anzahl und/oder der Dichte der vorgesehenen Kreuzungsstellen, desto höher ist die erreichbare Messempfindlichkeit der Fäden.

Vorzugsweise weisen die elektrisch leitfähigen Fäden mindestens 10 Kreuzungsstellen, vorzugsweise mindestens 25 Kreuzungsstellen, vorzugsweise mindestens 50 Kreuzungsstellen, vorzugsweise mindestens 75 Kreuzungsstellen auf.

Vorzugsweise weisen die elektrisch leitfähigen Fäden einen elektrisch leitfähigen Kern auf. Ferner weist vorzugsweise mindestens einer der elektrisch leitfähigen Fäden eine Umhüllung auf, die den elektrisch leitfähigen Kern umhüllt. Die elektrisch leitfähigen Kerne und die mindestens eine Umhüllung bilden vorzugsweise zusammen einen Stromkreis.

In einer vorteilhaften Ausgestaltung kann die proximale Schicht mindestens eine elektrisch leitfähige Folie und mindestens einen elektrisch leitfähigen Faden aufweisen, deren elektrischer Widerstand durch den Druckeinfluss veränderbar ist. Die elektrisch leitfähige Folie und der elektrisch leitfähige Faden können Teil eines elektrischen Stromkreises sein, dessen elektrischer Widerstand durch den Druckeinfluss veränderbar ist.

Die elektrisch leitfähige Folie und/oder der elektrisch leitfähige Faden können durch eine Umhüllung, vorzugsweise aus Polymer, eingehüllt sein. Die Umhüllung kann vorzugsweise eine zumindest geringe elektrische Leitfähigkeit aufweisen.

Dabei können der elektrisch leitfähige Faden und die elektrisch leitfähige Folie in der proximalen Schicht mit geringem oder keinem Abstand zueinander angeordnet werden. Vorzugsweise können der elektrisch leitfähige Faden und die elektrisch leitfähige Folie sich kreuzen oder zumindest überlagern.

Durch eine Druckeinwirkung kann der Abstand zwischen zumindest einem Bereich des elektrisch leitfähigen Fadens und der elektrisch leitfähigen Folie reduziert werden, vorzugsweise durch Kompression der Umhüllung. Dadurch sinkt der elektrische Widerstand des Stromflusses zwischen den beiden leitfähigen Elementen, die effektiv eine Stromleitung bilden. Die Veränderung des elektrischen Widerstands kann gemessen werden, um daraus ein Drucksignal generieren zu können.

In einer vorteilhaften Ausgestaltung kann die proximale Schicht mindestens zwei elektrisch leitfähige Folien aufweisen, deren elektrischer Widerstand durch den Druckeinfluss veränderbar ist. Es ist somit denkbar, gänzlich auf elektrisch leitfähige Fäden zu verzichten und stattdessen zwei elektrisch leitfähige Folien zu verwenden.

Zumindest eine der elektrisch leitfähigen Folien kann vorzugsweise durch eine Umhüllung, vorzugsweise aus Polymer, eingehüllt ist. Die beiden Folien können somit einen elektrischen Stromkreis bilden, dessen elektrischer Widerstand durch den Druckeinfluss veränderbar ist, wie bereits beschrieben.

Eine derartige elektrisch leitfähige Folie kann beispielsweise eine dünne Flächenschicht sein mit einer Dicke, die vorzugsweise kleiner als 5 mm, vorzugsweise kleiner als 3 mm, vorzugsweise kleiner als 2 mm ist.

Die elektrisch leitfähige Folie kann beispielsweise eine metallische Folie aufweisen, beispielsweise aus Kupfer. Denkbar ist jedoch auch eine elektrisch leitfähige Folie aus leitfähigem Textil.

Vorzugsweise kann das Blutdruckmessgerät zusätzlich eine Strommesseinrichtung zum Messen einer Stromstärke umfassen, wobei zumindest ein Bereich des druckempfindlichen Elements mit wenigstens einer Spannungsquelle verbunden ist und das Drucksignal aus den mittels der Strommesseinrichtung gemessenen Stromstärken generierbar ist. Mittels der Strommessung kann eine Veränderung des elektrischen Widerstands des druckempfindlichen Elements, basierend auf dem bereits beschriebenen Effekt, gemessen werden, um daraus ein Drucksignal zu generieren.

Das Blutdruckmessgerät kann alternativ oder zusätzlich zu dem druckempfindlichen Element der proximalen Schicht mindestens einen weiteren Drucksensor umfassen. Dieser zusätzliche Drucksensor kann vorzugsweise ein piezoelektrisches Element sein. Mittels des piezoelektrischen Elements können Druckeinwirkungen, die durch den arteriellen Blutdruck bewirkt werden, präzise erfasst werden. Alternativ oder zusätzlich kann der zusätzliche Drucksensor ein oder mehrere Dehnungsmessstreifen und/oder ein oder mehrere druckempfindliche Halbleiterchips und/oder ein oder mehrere Hall-Elemente sein.

Denkbar ist auch eine Vielzahl von piezoelektrischen Elementen, die vorzugsweise verteilt angeordnet sind.

Das Blutdruckmessgerät kann hierzu zusätzlich eine Spannungsmesseinrichtung zum Messen einer Spannungsänderung umfassen, die durch den Druckeinfluss auf das mindestens eine piezoelektrische Element bewirkt wird. Durch die Druckeinwirkung auf das piezoelektrische Element wird eine Ladungsänderung hervorgerufen, die mittels der Spannungsmesseinrichtung erfassbar ist. Aus der erfassten Spannungsänderung kann ein Drucksignal erzeugt werden.

Vorzugsweise ist die distale Schicht aus einem Faserverbundwerkstoff gebildet, der hochfeste Fasern umfasst. Die hochfesten Fasern können vorzugsweise Aramidfasern, beispielsweise Kevlar^{®}, umfassen, die vorzugsweise geflechtartig angeordnet sind. Denkbar sind jedoch auch alternative hochfeste Fasern, die ein Elastizitätsmodul von vorzugsweise mindestens 50.000 N/mm², bevorzugter mindestens 75.000 N/mm², bevorzugter mindestens 100.000 N/mm², besonders bevorzugt mindestens 125.000 N/mm² aufweisen.

In einer vorteilhaften Ausgestaltung weist die Messmanschette eine Spannvorrichtung zum Spannen der Messmanschette wenigstens bereichsweise um den Umfang der Extremität des Patienten auf.

Die Spannvorrichtung kann insbesondere eine manuell durch den Patienten betätigbare Spannvorrichtung sein. Denkbar ist jedoch auch eine automatische Spannvorrichtung, welche die Spannkraft nach Betätigung eines Betätigungselements durch den Patienten an der Spannvorrichtung selbsttätig erzeugen kann.

Ferner kann die Spannvorrichtung einen Schnelllösemechanismus aufweisen. Dieser Schnelllösemechanismus kann ein Lösebetätigungselement aufweisen, welches durch den Patienten betätigbar ist, um anschließend selbsttätig die Spannkraft der Spannvorrichtung zu reduzieren.

Vorzugsweise ist die Spannvorrichtung dazu eingerichtet, einen Anlegedruck an der Extremität des Patienten zu begrenzen. Die Begrenzung des Anlegedrucks kann durch den Patienten oder durch medizinisches Fachpersonal vordefiniert einstellbar sein. Die Begrenzung kann insbesondere mechanisch ausgebildet sein, beispielsweise in Form eines mechanischen Stopps. Ein derartiger mechanischer Stopp kann eine weitere Anspannung der Messmanschette durch die Spannvorrichtung verhindern. Der mechanische Stopp ist vorzugsweise individuell einstellbar.

Im Falle einer automatisierten Spannvorrichtung ist jedoch eine digitale Begrenzung ebenfalls denkbar. In diesem Fall wird der Anspannvorgang durch die automatisierte Spannvorrichtung beendet, nachdem ein vordefinierter Grenzwert eines Anlegedrucks erreicht wurde.

Der Anlegedruck kann insbesondere von dem Anteil des Weichteilgewebes des Körpers des Patienten an der entsprechenden Anlegestelle der Messmanschette abhängig sein. Je höher der Anteil des Weichteilgewebes desto höher sollte der einzustellende Anlegedruck der Messmanschette grundsätzlich sein.

Der Anteil des Weichteilgewebes hängt zum einen von der allgemeinen körperlichen Zusammensetzung, insbesondere von dem entsprechenden Fettanteil, des Patienten ab. Außerdem variiert der Anteil des Weichteilgewebes je nach Körperregion des Menschen. So weist beispielsweise der Unterarm eines Menschen einen geringeren Anteil von Weichteilgewebe auf als der Oberarm. Deshalb ist der Unterarm des Patienten in der Regel eine geeignetere Anlegestelle für die Messmanschette als der Oberarm.

Das Blutdruckmessgerät kann entsprechend eine Eingabemöglichkeit aufweisen, beispielsweise an der Auswertevorrichtung, mittels welcher eine Eingabe der allgemeinen körperlichen Zusammensetzung des entsprechenden Patienten, beispielsweise in Form eines Body-Mass-Indexes (BMI) oder eines Körperfettgehalts, ermöglicht, um den minimalen und/oder maximalen Anlegedruck der Messmanschette zu bestimmen. Außerdem kann das Blutdruckmessgerät mittels einer Anzeige, beispielsweise an der Auswertevorrichtung, dem Patienten eine empfohlene Anlegestelle der Messmanschette anzeigen, beispielsweise am Unterarm oder am Oberarm des Patienten, beispielsweise in Abhängigkeit von der Eingabe der allgemeinen körperlichen Zusammensetzung des entsprechenden Patienten oder in Abhängigkeit einer Vorgabe eines medizinisches Personals.

Denkbar ist, dass die Spannvorrichtung dazu eingerichtet sein kann, mittels einer Steuereinrichtung elektronisch, beispielweise durch den Patienten selbst, gesteuert zu werden. Insbesondere können die Steuereinrichtung und die Spannvorrichtung dazu eingerichtet sein, kabellos miteinander zu kommunizieren, beispielsweise mittels Bluetooth, Wifi oder Near-Field-Communication (NFC). Somit kann beispielsweise der Anlegedruck der Messmanschette an der Extremität des Patienten durch Betätigung der Steuereinrichtung erhöht oder reduziert werden. Dadurch kann das Anlegen der Messmanschette an einer Extremität des Patienten, insbesondere an einem Arm des Patienten, erleichtert werden. Somit kann der Patient die Messmanschette selbst anlegen und den entsprechenden Anlegedruck selbst einstellen, ohne dabei auf eine weitere Person angewiesen zu sein.

Die Steuereinrichtung kann Teil des Blutdruckmessgeräts sein. Denkbar ist jedoch auch die Verwendung einer externen Steuereinrichtung, beispielsweise durch ein Smartphone oder ein Tablet. Hierzu kann die externe Steuereinrichtung, beispielsweise ein Smartphone oder ein Tablet, ein Softwareprogramm aufweisen, welches eine Kommunikation mit der Spannvorrichtung ermöglicht.

Die Spannvorrichtung kann beispielsweise einen Antrieb, vorzugsweise einen elektrischen Antrieb, aufweisen, der dazu eingerichtet ist, den Anlegedruck der Messmanschette zu erhöhen oder zu reduzieren. Der Antrieb kann beispielsweise mittels einer Steuereinrichtung, beispielsweise in der Gestalt der oben beschriebenen Steuereinrichtung, gesteuert werden.

Alternativ oder zusätzlich kann der Antrieb auch automatisch beim Anlegen der Messmanschette an der Extremität des Patienten aktivierbar sein. Beispielseise kann die Messmanschette einen oder mehrere Sensoren aufweisen, die einen angelegten Zustand der Messmanschette detektieren können, um den Antrieb zu aktivieren und den Anlegedruck der Messmanschette einzustellen.

Hierzu kann die Spannvorrichtung ein Greifelement, vorzugsweise ein Greifrad, aufweisen, welches von dem Antrieb angetrieben werden kann. Das Greifelement kann dazu eingerichtet sein, zumindest einen Bereich der Messmanschette oder eine mit der Messmanschette verbundene Struktur zu greifen und vorzugsweise eine Zugkraft darauf auszuüben, um die Messmanschette an der Extremität des Patienten zu spannen.

Hierzu kann vorzugsweise der Bereich der Messmanschette oder die mit der Messmanschette verbundene Struktur, an der bzw. an dem das Greifelement angreift, Strukturen aufweisen, beispielsweise in Form von Vertiefungen oder Vorsprünge, die das Greifen mittels des Greifelements erleichtert. Das Greifelement kann insbesondere ein Zahnrad sein, welches beispielweise mit den Vertiefungen oder Vorsprüngen in Eingriff steht und mit diesen zusammenarbeiten kann, um die Messmanschette an der Extremität des Patienten zu spannen.

Vorzugsweise ist die Spannvorrichtung dazu eingerichtet ist, den Anlegedruck in Abhängigkeit von dem erzeugten Drucksignal zu begrenzen. Vorzugsweise wird der Anlegedruck auf einen Druckwert begrenzt, der unterhalb des systolischen Blutdrucks liegt, bevorzugt unterhalb des diastolischen Blutdrucks.

Dadurch kann der Anlegedruck der Messmanschette auf einen vordefinierten Wert präzise eingestellt werden. Außerdem bewirkt ein Anlegedruck der Messmanschette, der unterhalb des systolischen Blutdrucks bzw. unterhalb des diastolischen Blutdrucks liegt, einen höheren Tragekomfort im Vergleich zu eingangs beschriebenen konventionellen Blutdruckmessgeräten.

In einer vorteilhaften Ausgestaltung weist die Spannvorrichtung einen Wickeldorn auf, um den zumindest ein Bereich der Messmanschette aufwickelbar ist. Vorzugsweise ist der Wickeldorn ausgebildet, zwei Bereiche der Messmanschette gegenläufig aufzuwickeln.

Denkbar ist jedoch auch ein Klettverschluss als Spannvorrichtung. In diesem Fall kann der Klettverschluss eine Referenzlinie als Soll-Spannstelle aufweisen, an der sich der Patient oder das medizinische Fachpersonal orientieren kann, um einen vordefinierten Anlegedruck der Messmanschette zu realisieren.

Vorzugsweise kann der Wickeldorn ein Arretiermittel aufweisen, welches eine Drehung des Wickeldorns in eine entgegen der Wickelrichtung gerichtete Richtung verhindert. Dadurch kann ein ungewolltes Lösen der Spannvorrichtung verhindert werden.

Zum Lösen der Spannvorrichtung kann der Wickeldorn ein Auslöseelement aufweisen, welches die Wirkung des Arretiermittels aufhebt, sodass der Wickeldorn in die entgegen der Wickelrichtung gerichtetete Richtung drehbar ist, um die Spannung der Messmanschette an der Extremität des Patienten zu lösen.

Vorzugsweise kann die Messmanschette derart ausgebildet und eingerichtet sein, dass der Anlegedruck der Messmanschette während eines Messvorgangs konstant bleibt. Dadurch kann eine zuverlässige Messung des Blutdrucks über einen längeren Zeitraum sichergestellt werden.

In einer vorteilhaften Ausgestaltung umfasst das Blutdruckmessgerät vorzugsweise zusätzlich mindestens eine Messelektrode, die dazu eingerichtet ist, an dem Patienten angelegt zu werden und Muskelaktivitäten, vorzugsweise von einer Vielzahl von Muskeln, des Patienten zu erfassen und an die Auswertevorrichtung bereitzustellen. Die Erfassung erfolgt vorzugsweise mittels elektrischer Myographie.

Die Messelektrode kann vorzugsweise dazu eingerichtet sein, an der Extremität des Patienten angebracht zu werden, an der die Messmanschette angelegt ist. Dadurch können Muskelkontraktion, die beispielsweise zum Bewegen von Teilen des menschlichen Körpers dienen, erfasst werden.

Die Messelektrode kann hierzu Befestigungsmittel aufweisen, beispielsweise in Form eines Saugnapfes oder eines Klebematerials.

Die Messelektrode kann mittels eines Verbindungskabels mit der Auswertevorrichtung verbunden sein. Denkbar ist jedoch auch eine kabellose Verbindung der Messelektrode zu der Auswertevorrichtung, beispielsweise mittels Bluetooth, Wifi oder Near-Field-Communication (NFC).

Denkbar ist auch eine Vielzahl von Messelektroden, die an unterschiedlichen Stellen des Körpers des Patienten angebracht werden können.

In einer vorteilhaften Ausgestaltung ist die Auswertevorrichtung dazu eingerichtet, den Blutdruck des Patienten zusätzlich in Abhängigkeit von den erfassten elektrischen Muskelaktivitäten zu bestimmen.

Dadurch können Muskelkontraktionen, die eine Druckeinwirkung auf die Messmanschette bewirken können, beim Bestimmen des Blutdrucks berücksichtigt werden, sodass diese bei der Auswertung des Drucksignals durch die Auswerteeinrichtung keine oder zumindest nur eine geringe Auswirkung auf den bestimmten Blutdruck haben. Dadurch kann der tatsächliche Blutdruck des Patienten präziser und unabhängig von eventuellen Muskelkontraktionen im Bereich der angelegten Messmanschette bestimmt werden.

In einer vorteilhaften Ausgestaltung umfasst das Blutdruckmessgerät zusätzlich mindestens eine weitere Messelektrode. Die weitere Messelektrode kann insbesondere dazu eingerichtet sein, an dem Patienten angelegt zu werden und Atmungszustände des Patienten zu erfassen und an die Auswertevorrichtung bereitzustellen. Dadurch kann beispielsweise die Atemfrequenz und/oder das Atemvolumen pro Atemvorgang erfasst werden. Da die Atemaktivität des Patienten Einfluss auf den Blutdruck hat, kann so der jeweilige Atemzustand in die Berechnung des Blutdrucks einfließen und somit das Messergebnis verbessern.

Eine derartige Messelektrode zum Erfassen von Atmungszuständen des Patienten kann beispielswiese eine Messelektrode sein, welche Bewegungen des Brustkorbs beim Ein- und Ausatmen des Patienten erfasst. Ebenfalls denkbar ist beispielsweise eine Vorrichtung zum Messen von Strömungsgeräuschen in der Lunge, beispielsweise in Form eines Stethoskops, um Atmungszustande des Patienten zu erfassen. Ebenso ist es möglich, Muskelkontraktionen zu messen, die Rückschlüsse auf den Atemzustand erlauben.

Denkbar ist auch das Vorsehen einer Vielzahl von Messelektroden zum Erfassen von Atmungszuständen des Patienten.

Vorzugsweise kann die Auswertevorrichtung dazu eingerichtet sein, den Blutdruck des Patienten zusätzlich in Abhängigkeit von dem erfassten Atmungszustand zu bestimmen.

Änderungen der Druckverhältnisse im Brustkorb treten während des Atmens auf, was wiederum das erzeugte Drucksignal beeinflusst. Derartige Einflüsse sind jedoch unerwünscht, da lediglich der Blutdruck, möglichst isoliert von anderen Druckeinflüssen auf die Messmanschette, bestimmt werden soll.

Um diesen Einfluss in dem erzeugten Drucksignal zu korrigieren, kann die Auswertevorrichtung dazu eingerichtet, diesen Einfluss anhand des erfassten Atmungszustands zu isolieren und zu entfernen, sodass der Einfluss bei der Auswertung des Drucksignals durch die Auswerteeinrichtung keine oder zumindest nur eine geringe, rechnerisch korrigierbare Auswirkung auf den bestimmten Blutdruck haben.

In einer vorteilhaften Ausgestaltung kann das Blutdruckmessgerät zusätzlich eine Herzfrequenz-Messvorrichtung umfassen. Die Herzfrequenz-Messvorrichtung kann dazu eingerichtet sein, an dem Patienten angelegt zu werden und die Herzfrequenz des Patienten zu erfassen und ein Signal an die Auswertevorrichtung bereitzustellen.

Eine derartige Herzfrequenz-Messvorrichtung kann beispielsweise unmittelbar die Herzschläge des Patienten detektieren, beispielsweise in der Gestalt eines Elektrokardiogramms (EKG), um die Herzfrequenz, d.h. die Anzahl der Herzschläge pro Minute, zu bestimmen.

Denkbar sind jedoch auch Messvorrichtungen, welche die Herzfrequenz indirekt bestimmen, beispielsweise mittels optischer Messung. Hierzu kann die Herzfrequenz-Messvorrichtung eine Lichtquelle zum Emittieren von Licht auf einen Körperbereich des Patienten, vorzugsweise am Handgelenk oder am Finger des Patienten, aufweisen. Ferner kann die Herzfrequenz-Messvorrichtung eine Vorrichtung zum Messen eines von dem Körperbereich des Patienten reflektierten Anteils des Lichts aufweisen. Auf Grund des Pulsierens der Blutgefäße variiert der Anteil des durch das Blut absorbierten Anteils des Lichts in zeitlicher Hinsicht. Aus der Erfassung dieser zeitlich variierenden Reflektions- und Absorbtionsanteile des Lichts kann die Herzfrequenz des Patienten bestimmt werden.

Vorzugsweise kann die Auswertevorrichtung ferner dazu eingerichtet sein, den Blutdruck des Patienten zusätzlich in Abhängigkeit einer Herzfrequenz des Patienten zu bestimmen.

Dadurch kann der Einfluss der Herzfrequenz auf das Drucksignal identifiziert und zumindest teilweise entfernt werden, sodass die Herzfrequenz keine oder zumindest nur eine geringe Auswirkung auf den bestimmten Blutdruck hat.

In einer vorteilhaften Ausgestaltung kann die Auswertevorrichtung als Teil der Messmanschette, insbesondere in einer der distalen oder proximalen Schicht oder in einer zusätzlichen Schicht, ausgebildet sein.

Dadurch kann das Blutdruckmessgerät kompakt gebaut werden, wodurch die Beeinträchtigung des Patienten beim Tragen des Blutdruckmessgeräts reduziert wird.

In einer vorteilhaften Ausgestaltung kann das Blutdruckmessgerät zusätzlich eine Übertragungseinheit umfassen. Die Übertragungseinheit kann dazu eingerichtet sein, die Auswertesignale der Auswertevorrichtung kabellos, beispielsweise mittels Bluetooth, Wifi oder NFC, an eine Empfangseinheit zu übertragen.

Dadurch können die gemessenen Blutdruckwerte komfortabel und schnell dem Patienten bereitgestellt werden. Außerdem wird dadurch eine individualisierbare Auswertung der kontinuierlichen Blutdruckmessung an der Empfangseinheit, beispielsweise am Smartphone des Patienten, ermöglicht.

Des Weiteren ist der Patient zum Auslesen und Auswerten der gemessenen Blutdruckwerte nicht auf Fachpersonal angewiesen.

Die Empfangseinheit kann ein Teil des Blutdruckmessgeräts selbst sein. Die Verwendung eines externen Geräts, beispielsweise eines Smartphones oder eines Tablets, ist ebenfalls denkbar.

Denkbar ist auch eine Übertragung der gemessenen Blutdruckwerte an einen zentralen Speicher, beispielsweise an einen Server oder eine Cloud. Dort können die gemessenen Blutdruckwerte gespeichert und von dem Patienten abgerufen werden.

In einer vorteilhaften Ausgestaltung kann das Blutdruckmessgerät zusätzlich eine akustische Detektionsvorrichtung umfassen. Die Detektionsvorrichtung kann dazu eingerichtet sein, an dem Patienten angelegt zu werden und Geräusche, die durch Muskelkontraktion des Patienten verursacht werden, zu detektieren, vorzugsweise mittels akustischer Myographie, und ein Signal an die Auswertevorrichtung bereitzustellen.

Das Messprinzip mittels akustischer Myographie zur Detektion von Muskelkontraktionen ist beispielsweise in dem Artikel "Portable acoustic myography - a realistic noninvasive method for assessment of muscle activity and coordination in human subjects in most home and sports settings" (https://www.ncbi.nlm.nih.gov/pmc/articles/PMC3831924) beschrieben.

Vorzugsweise kann die Auswertevorrichtung dazu eingerichtet sein, den Blutdruck des Patienten zusätzlich in Abhängigkeit der detektierten Geräusche zu bestimmen.

Dadurch können Muskelkontraktionen, die eine Druckeinwirkung auf die Messmanschette bewirken können, beim Bestimmen des Blutdrucks berücksichtigt werden, sodass diese bei der Auswertung des Drucksignals durch die Auswerteeinrichtung keine oder zumindest nur eine geringe Auswirkung auf den bestimmten Blutdruck haben. Dadurch kann der tatsächliche Blutdruck des Patienten präziser und unabhängig von eventuellen Muskelkontraktionen im Bereich der angelegten Messmanschette bestimmt werden.

In einer vorteilhaften Ausgestaltung kann das Blutdruckmessgerät zusätzlich eine Bewegungsmessvorrichtung umfassen. Die Bewegungsmessvorrichtung kann dazu eingerichtet sein, Bewegungen des Patienten zu detektieren und ein Signal an die Auswertevorrichtung bereitzustellen. Vorzugsweise kann die Auswertevorrichtung dazu eingerichtet sein, den Blutdruck des Patienten zusätzlich in Abhängigkeit der detektierten Bewegungen des Patienten zu bestimmen.

Dadurch können über die Erfassung von Bewegungen des Körpers des Patienten indirekt Muskelkontraktion, die eine Druckeinwirkung auf die Messmanschette bewirken können, beim Bestimmen des Blutdrucks berücksichtigt werden, sodass diese bei der Auswertung des Drucksignals durch die Auswerteeinrichtung keine oder zumindest nur eine geringe Auswirkung auf den bestimmten Blutdruck haben. Dadurch kann der tatsächliche Blutdruck des Patienten präziser und unabhängig von eventuellen Muskelkontraktionen im Bereich der angelegten Messmanschette bestimmt werden.

Die Bewegungsmessvorrichtung kann vorzugsweise dazu eingerichtet sein, an einer Extremität des Patienten angebracht zu werden.

Vorzugsweise weist die Bewegungsmessvorrichtung eine kabellose Kommunikationsverbindung zu der Auswertevorrichtung auf. Denkbar ist jedoch auch eine Verbindung der Bewegungsmessvorrichtung zu der Auswertevorrichtung mittels eines Übertragungskabels zum Übertragen von Daten an die Auswertevorrichtung.

Die Bewegungsmessvorrichtung kann vorzugsweise einen Beschleunigungssensor oder ein Gyroskop aufweisen.

Denkbar ist auch die Verwendung eines externen Bewegungssensors, welcher nicht Teil des Blutdruckmessgeräts ist. Ein derartiger externer Bewegungssensor kann beispielsweise in dem Smartphone des Patienten angeordnet sein. In diesem Fall kann der externe Bewegungssensor eine, vorzugsweise kabellose, Kommunikationsverbindung zu der Auswertevorrichtung aufweisen.

Vorzugsweise kann die Bewegungsmessvorrichtung dazu eingerichtet sein, Bewegungen der Extremität des Patienten, an der die Messmanschette angelegt ist, relativ zum Rumpf des Patienten zu detektieren und ein Signal an die Auswertevorrichtung bereitzustellen. Vorzugsweise kann die Auswertevorrichtung dazu eingerichtet sein, den Blutdruck des Patienten zusätzlich in Abhängigkeit von den detektierten Bewegungen der Extremität des Patienten zu bestimmen.

Dadurch können Bewegungen einer Extremität, an der die Messmanschette des Blutdruckmessgeräts angelegt ist, isoliert von Bewegungen anderer Körperbereiche des Patienten erfasst werden. Dadurch werden nur diejenigen Muskelkontraktionen, die unmittelbar einen Einfluss auf das erzeugte Drucksignal haben, indirekt über die entsprechenden Bewegungen der Extremität erfasst und bei der Korrektur des Drucksignals mittels der Auswertevorrichtung berücksichtigt.

Die Bewegungsmessvorrichtung zum Detektieren von Bewegungen der Extremität des Patienten relativ zum Rumpf des Patienten kann beispielsweise durch das Vorsehen mindestens zweier Bewegungsmesssensoren realisiert werden, wobei mindestens ein Bewegungsmesssensor an dem Rumpf des Patienten und mindestens ein Bewegungsmesssensor an der entsprechenden Extremität des Patienten angebracht werden kann. Aus den erfassten Bewegungen beider Bewegungsmesssensoren, beispielsweise mittels Differenzbildung beider erfassten Bewegungswerte, beispielsweise mittels der Auswertevorrichtung, kann eine Bewegung der Extremität des Patienten, an der die Messmanschette angelegt ist, relativ zum Rumpf des Patienten detektiert werden. Vorzugsweise kann ein Bewegungsmesssensor in der Messmanschette integriert sein.

In einer vorteilhaften Ausgestaltung kann das Blutdruckmessgerät zusätzlich eine Empfangseinheit mit einer Anzeige umfassen. Vorzugsweise kann die Empfangseinheit dazu eingerichtet sein, die mittels des Blutdruckmessgeräts gemessenen Werte und den bestimmten Blutdruck von der Auswertevorrichtung zu empfangen und anzuzeigen.

Vorzugsweise kann die Empfangseinheit ein mobiles, vorzugsweise kabelloses, Gerät sein.

Die Offenlegung betrifft außerdem Verfahren zum kontinuierlichen, nicht-invasiven Bestimmen eines Blutdrucks eines Patienten. Die zu dem Blutdruckmessgerät beschriebenen Vorteile gelten ebenfalls für das nachfolgend beschriebene Verfahren, dass bevorzugt unter Verwendung einer vorstehend beschriebenen Manschette durchgeführt wird.

Das Verfahren kann folgende Schritte umfassen:
- Anlegen einer Messmanschette, vorzugsweise einer hierin beschriebenen Messmanschette, wenigstens bereichsweise um einen Umfang einer Extremität eines Patienten, wobei die Messmanschette wenigstens eine proximale Schicht und eine distale Schicht aufweist, wobei die proximale Schicht ein druckempfindliches Element aufweist und wobei die distale Schicht zumindest in Umfangsrichtung unter Druckeinfluss im Wesentlichen nicht dehnbar ist;
- Erzeugen eines Drucksignals durch Druckeinfluss auf das druckempfindliche Element, wobei das Drucksignal zu dem Druckeinfluss äquivalent und kontinuierlich ist;
- Bestimmen des Blutdrucks des Patienten mittels einer Auswertevorrichtung in Abhängigkeit von einem Drucksignal.

In einer vorteilhaften Ausgestaltung können zusätzlich Muskelaktivitäten, vorzugsweise von einer Vielzahl von Muskeln, des Patienten mittels mindestens einer Messelektrode erfasst werden, vorzugsweise mittels elektrischer Myographie, und der Blutdruck des Patienten zusätzlich in Abhängigkeit von den erfassten elektrischen Muskelaktivitäten bestimmt werden.

Ferner können vorzugsweise zusätzlich Atmungszustände des Patienten erfasst werden und der Blutdruck des Patienten zusätzlich in Abhängigkeit von dem erfassten Atmungszustand bestimmt werden.

Vorzugsweise können zusätzlich Geräusche, die durch Muskelkontraktion des Patienten verursacht werden, mittels mindestens einer akustischen Detektionsvorrichtung detektiert werden, vorzugsweise mittels akustischer Myographie, und der Blutdruck des Patienten zusätzlich in Abhängigkeit von den erfassten Geräuschen bestimmt werden.

Vorzugsweise kann zusätzlich die Herzfrequenz des Patienten mittels einer Herzfrequenz-Messvorrichtung erfasst werden und der Blutdruck des Patienten zusätzlich in Abhängigkeit von der erfassten Herzfrequenz bestimmt werden.

Vorzugsweise können zusätzlich Bewegungen des Patienten mittels einer Bewegungsmessvorrichtung detektiert werden und der Blutdruck des Patienten zusätzlich in Abhängigkeit von den detektierten Bewegungen bestimmt werden.

Vorzugsweise können zusätzlich mittels einer Bewegungsmessvorrichtung Bewegungen der Extremität des Patienten, an der die Messmanschette angelegt ist, relativ zum Rumpf des Patienten detektiert werden und der Blutdruck des Patienten zusätzlich in Abhängigkeit von den detektierten Bewegungen der Extremität des Patienten bestimmt werden.

Ferner können vorzugsweise die mittels des Blutdruckmessgeräts gemessenen Werte und der bestimmte Blutdruck von der Auswertevorrichtung an eine Empfangseinheit mit einer Anzeige gesendet werden, wobei die Empfangseinheit vorzugsweise ein mobiles externes Gerät ist, vorzugsweise ein Smartphone oder ein Tablet. Vorzugsweise kann die Übertragung der gemessenen Werte kabellos erfolgen, beispielsweise mittels Bluetooth, Wifi oder NFC.

Denkbar ist auch eine Übertragung der gemessenen Blutdruckwerte an einen zentralen Speicher, beispielsweise an einen Server oder an eine Cloud. Dort können die gemessenen Blutdruckwerte gespeichert und/oder ausgewertet werden und von dem Patienten abgerufen werden.

Weitere Vorteile, Merkmale und Einzelheiten der Erfindung ergeben sich aus der nachfolgenden Beschreibung eines bevorzugten Ausführungsbeispiels sowie anhand der Zeichnungen.
- Fig. 1: zeigt in einer schematischen perspektiven Ansicht ein Ausführungsbeispiel eines erfindungsgemäßen Blutdruckmessgeräts;
- Fig. 2: zeigt in einer schematischen perspektiven Ansicht ein weiteres Ausführungsbeispiel eines erfindungsgemäßen Blutdruckmessgeräts;
- Fig. 3: zeigt in einer Querschnittsansicht ein erfindungsgemäßes Blutdruckmessgerät im angelegten Zustand;
- Fig. 4: zeigt in einer schematischen perspektiven Ansicht die proximale Schicht des Blutdruckmessgeräts aus Fig. 1 und 2;
- Fig. 5: zeigt in einer schematischen perspektiven Ansicht ein weiteres Ausführungsbeispiel eines erfindungsgemäßen Blutdruckmessgeräts;
- Fig.6: zeigt in einer schematischen perspektiven Ansicht ein weiteres Ausführungsbeispiel eines erfindungsgemäßen Blutdruckmessgeräts;
- Fig. 7: zeigt in einer schematischen perspektiven Ansicht einen elektrisch leitfähigen Faden eines erfindungsgemäßen Blutdruckmessgeräts;
- Fig. 8: zeigt ein Messprinzip eines erfindungsgemäßen Blutdruckmessgeräts;
- Fig. 9: zeigt ein Messdiagramm einer Messung mit einem erfindungsgemäßen Blutdruckmessgerät;
- Fig. 10: zeigt ein Messdiagramm einer Messung mit einem erfindungsgemäßen Blutdruckmessgerät;
- Fig. 11: zeigt ein Messdiagramm einer Messung mit einem erfindungsgemäßen Blutdruckmessgerät;
- Fig. 12: zeigt ein weiteres Messdiagramm einer Messung mit einem erfindungsgemäßen Blutdruckmessgerät;
- Fig. 13: zeigt ein weiteres Messdiagramm einer Messung mit einem erfindungsgemäßen Blutdruckmessgerät;
- Fig. 14: zeigt ein weiteres Messdiagramm einer Messung mit einem erfindungsgemäßen Blutdruckmessgerät;
- Fig. 15: zeigt ein weiteres Messdiagramm einer Messung mit einem erfindungsgemäßen Blutdruckmessgerät;
- Fig. 16: zeigt ein weiteres Messdiagramm einer Messung mit einem erfindungsgemäßen Blutdruckmessgerät;
- Fig. 17: zeigt ein weiteres Messdiagramm einer Messung mit einem erfindungsgemäßen Blutdruckmessgerät; und
- Fig. 18: zeigt ein weiteres Messdiagramm einer Messung mit einem erfindungsgemäßen Blutdruckmessgerät.

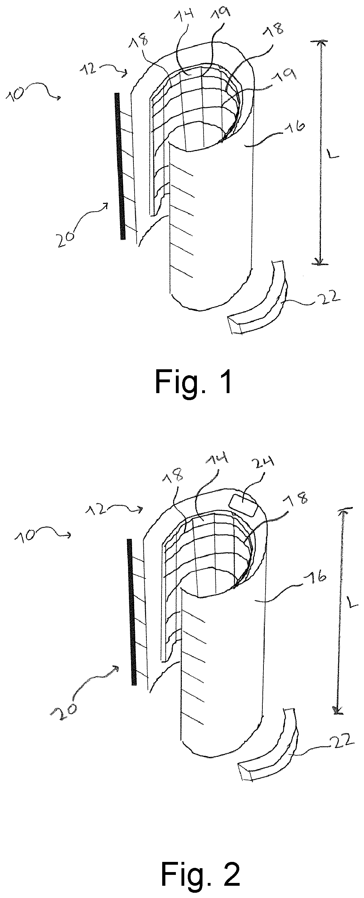

Figur 1 zeigt ein Blutdruckmessgerät 10 mit einer Messmanschette 12. Die Messmanschette 12 weist eine proximale Schicht 14 und eine distale Schicht 16 auf.

Die proximale Schicht 14 weist druckempfindliche Elemente 18 in Form von elektrisch leitfähigen Fäden 18 auf. Die elektrisch leitfähigen Fäden 18 bilden Kreuzungsstellen 19, an denen sich zwei verschiedene elektrisch leitfähige Fäden 18 kreuzend überlagern. Zumindest einer der elektrisch leitfähigen Fäden 18 weist eine Umhüllung, vorzugsweise aus Polymer, auf, die einen elektrisch leitfähigen Kern umhüllt. Die Umhüllung kann vorzugsweise druckempfindlich, vorzugsweise komprimierbar, sein

Alternativ kann anstatt zumindest eines der elektrisch leitfähigen Fäden 18 eine elektrische leitfähige Folie vorgesehen sein. Auch beide elektrisch leitfähige Fäden 18 können jeweils durch eine elektrisch leitfähige Folie ersetzt werden. Alternativ oder zusätzlich kann ein piezoelektrisches Material, Dehnungsmessstreifen oder druckempfindliche Halbleiterchips oder Hall-Elemente vorgesehen sein.

Die distale Schicht 16, welche die proximale Schicht 14 vollständig umgreift, ist zumindest in Umfangsrichtung unter Druckeinfluss im Wesentlichen nicht dehnbar. Vorzugsweise weist die distale Schicht hierzu hochfeste Fasern, vorzugsweise Aramidfasen, auf.

Die Messmanschette 12 ist derart dimensioniert und eingerichtet, wenigstens bereichsweise um einen Umfang einer Extremität eines Patienten angelegt zu werden. Im angelegten Zustand ist die proximale Schicht 14 näher an der Extremität des Patienten angeordnet als die distale Schicht 16.

Die proximale Schicht 14 mit den elektrisch leitfähigen Fäden 18 ist somit auf Grund der umgebenden nicht dehnbaren distalen Schicht 16 zumindest in radialer Richtung unbeweglich fixiert, sodass eine radiale Auslenkung der proximalen Schicht 14 durch einen Druckeinfluss von der Extremität des Patienten auf die proximale Schicht im angelegten Zustand verhindert oder zumindest minimiert werden kann.

Das Blutdruckmessgerät 10 weist ferner eine Spannvorrichtung 20 auf, mit der die Messmanschette 12 an der Extremität des Patienten gespannt werden kann, um ein sicheres Anliegen der Messmanschette 12 an der Extremität des Patienten zu ermöglichen.

Das Blutdruckmessgerät 10 umfasst ferner eine Spannungsquelle (nicht abgebildet), welche die elektrisch leitfähigen Fäden 18 mit Strom versorgt. Die Spannungsquelle kann vorteilhaft ein kompaktes mobiles Gerät sein, sodass der Patient die Spannungsquelle bequem mit sich tragen kann.

Des Weiteren kann das Blutdruckmessgerät 10 ein Strommessgerät (nicht abgebildet) aufweisen, welches den durch die elektrisch leitfähigen Fäden fließenden Strom messen kann.

Außerdem weist das Blutdruckmessgerät 10 eine Auswertevorrichtung 22 auf, die dazu eingerichtet ist, in Abhängigkeit von dem Drucksignal den Blutdruck des Patienten zu bestimmen.

In Figur 1 weist die Auswertevorrichtung 22 eine drahtlose Verbindung zu den elektrisch leitfähigen Fäden 18 auf. Hierzu kann das Blutdruckmessgerät eine Übertragungseinheit (nicht dargestellt) aufweisen, die kabellos Signale von der Messmanschette 12 an die Auswertevorrichtung 22 übertragen kann. Denkbar ist jedoch auch das Vorsehen einer Kabelverbindung zwischen der Auswertevorrichtung 22 und den elektrisch leitfähigen Fäden 18 zur Übertragung des Drucksignals.

Die Auswertevorrichtung 22 kann außerdem ein integriertes Display aufweisen, auf dem die Blutdruckwerte für den Patienten sichtbar angezeigt werden. Alternativ kann die Auswertevorrichtung 22 dazu eingerichtet sein, die ermittelten Blutdruckwerte an ein externes Gerat, vorzugsweise mit einem Display, zu übertragen.

Im angelegten Zustand der Messmanschette 12 erfassen die elektrisch leitfähigen Fäden 18 die Druckeinwirkung des arteriellen Blutdrucks des Patienten, indem die Umhüllung zumindest eines der elektrisch leitfähigen Fäden 18 durch die Druckeinwirkung insbesondere an den Kreuzungsstellen 19 komprimiert wird. Dadurch sinkt der elektrische Widerstand der elektrisch leitfähigen Fäden 18. Die dadurch bewirkte Änderung des elektrischen Widerstands der elektrisch leitfähigen Fäden 18 kann mittels des Strommessgeräts erfasst werden.

Aus der erfassten Änderung des elektrischen Widerstands der elektrische leitfähigen Fäden 18 kann ein diesem Druckeinfluss äquivalentes, kontinuierliches Drucksignal erzeugt und an die Auswertevorrichtung 22 bereitgestellt werden. Die Auswertevorrichtung 22 kann in Abhängigkeit von dem bereitgestellten Drucksignal den Blutdruck des Patienten bestimmen.

Im Gegensatz zu konventionellen Blutdruckmessgeräten, kann das hierin beschriebene Blutdruckmessgerät 10 mit einem wesentlich geringeren Anlegedruck an der Extremität des Patienten angelegt werden. Insbesondere kann der Anlegedruck unterhalb des systolischen und/oder des diastolischen Blutdrucks liegen. Dadurch wird ein angenehmer Tragekomfort für den Patienten realisiert.

Der gewünschte Anlegedruck der Messmanschette 12 kann mittels des erzeugten Drucksignals selbst bestimmt werden, indem die Messmanschette 12 angelegt wird, während die Druckwerte entsprechend abgelesen werden. Bei Erreichen eines vorbestimmten Druckwerts kann der Anspannvorgang beendet werden.

Alternativ kann der Anlegedruck derart bestimmt werden, dass die Messmanschette 12 bis kurz vor dem Zustand fest gespannt wird, in dem sich ein sichtbarer Blutstau in der Handrückenvene bildet. Bei diesem Vorgang wird die Hand auf Höhe des Herzens gehalten.

Figur 2 zeigt ein weiteres Ausführungsbeispiel des Blutdruckmessgeräts 10. Dabei umfasst das in Figur 2 gezeigte Blutdruckmessgerät im Wesentlichen die Merkmale des Blutdruckmessgeräts 10 aus Figur 1.

Zusätzlich weist das Blutdruckmessgerät 10 gemäß dem Ausführungsbeispiel der Figur 2 mindestens ein Messinstrument 24 auf. Im vorliegenden Ausführungsbeispiel ist das Messinstrument 24 an der Messmanschette 12 angeordnet. Denkbar ist jedoch auch, das Messinstrument 24 direkt an dem Körper, beispielsweise an einer Extremität, oder an einem Kleidungsstück des Patienten anzubringen. Hierzu kann das Messinstrument 24 ein Befestigungsmittel aufweisen, beispielsweise in Form eines Saugnapfes oder eines Klebemittels, um das Messinstrument 24 an dem Patienten anzubringen.

Das Messinstrument 24 kann eine Elektrode sein, die dazu eingerichtet sein kann, an dem Patienten angelegt zu werden und Muskelaktivitäten des Patienten zu erfassen. Dies kann vorzugsweise mittels elektrischer Myographie erfolgen. Das erzeugte Signal der Elektrode 24 kann an die Auswertevorrichtung 22 bereitgestellt werden. Das erzeugte Signal kann dazu dienen, den Einfluss der detektierten Muskelaktivitäten auf das mittels der elektrisch leitfähigen Fäden 18 erzeugte Drucksignal zu isolieren und aus dem Drucksignal zu korrigieren.

Dadurch können Muskelkontraktionen, die eine Druckeinwirkung auf die Messmanschette 12 bewirken können, beim Bestimmen des Blutdrucks berücksichtigt werden, sodass diese bei der Auswertung des Drucksignals durch die Auswerteeinrichtung 22 keine oder zumindest nur eine geringe Auswirkung auf den bestimmten Blutdruck haben. Dadurch kann der tatsächliche Blutdruck des Patienten präziser und unabhängig von eventuellen Muskelkontraktionen im Bereich der angelegten Messmanschette 12 bestimmt werden.

Ferner kann das Messinstrument 24 ein Bewegungssensor sein, Bewegungen des Patienten, vorzugsweise der Extremität, an der die Messmanschette 12 angelegt ist, zu detektieren.

Ferner kann ein solcher Bewegungssensor 24 dazu dienen, Bewegungen des Brustkorbs des Patienten zu detektieren. Aus den detektieren Bewegungen des Brustkorbs kann die Atmung, beispielsweise die Atmungsfrequenz, des Patienten bestimmt werden.

Ferner kann das Messinstrument 24 eine Herzfrequenz-Messvorrichtung sein, die dazu eingerichtet ist, die Herzfrequenz des Patienten zu bestimmen.

Denkbar ist auch eine Kombination der oben beschriebenen Messinstrumente 24, um alle hierin beschriebenen Einflüsse auf das Drucksignal, die nicht durch den arteriellen Blutdruck des Patienten hervorgerufen werden, zu isolieren und in dem Drucksignal entsprechend zu korrigieren. Hierzu kann das Blutdruckmessgerät 10 eine Vielzahl unterschiedlicher Messinstrumente 24 aufweisen.

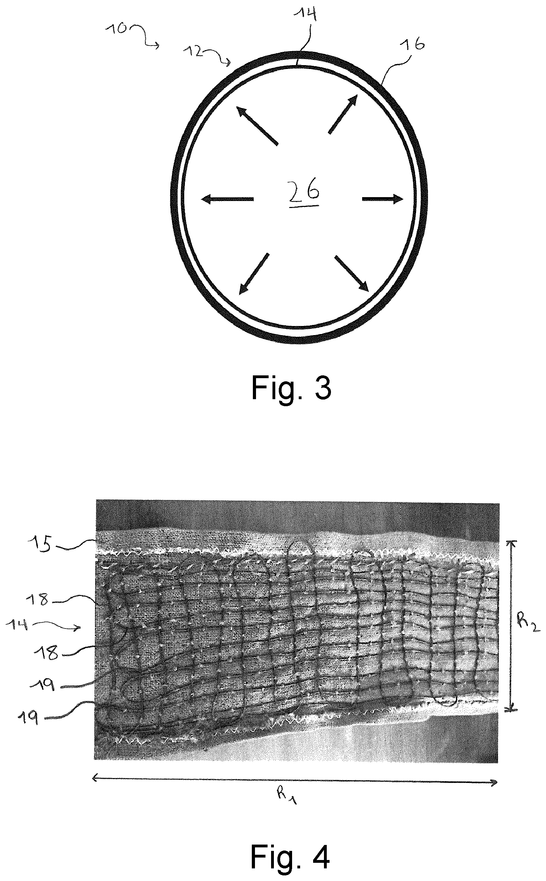

Figur 3 zeigt die Messmanschette 12 des Blutdruckmessgeräts 10 im angelegten Zustand an einer Extremität 26 eines Patienten. Dabei liegt die Messmanschette 12 umfassend die proximale Schicht 14 und die distale Schicht 16 an der Extremität 26 des Patienten an.

Pulsationen der Arterien in der Extremität 26 bewirken periodische radiale Ausdehnungen der Extremität 26. Diese radialen Ausdehnungen sind in Figur 3 mit radial nach außen gerichteten Pfeilen gekennenzeichnet.

Diese radialen Ausdehnungen werden mittels der druckempfindlichen Elemente 18 der proximalen Schicht 14 erfasst, wie vorstehend beschrieben.

In Figur 3 hüllt die Messmanschette 12 die Extremität 26 vollständig in Umfangsrichtung ein. Dies ist jedoch nur eine beispielhafte Darstellung. Es versteht sich, dass die Messmanschette 12 auch nur einen Bereich des Umfangs der Extremität 26 bedecken kann, beispielsweise über einen Umfangsbereich von 25% bis 90% der Extremität 26.

Figur 4 zeigt eine schematische perspektivische Ansicht einer beispielhaften proximalen Schicht 14 der Messmanschette 12. Die proximale Schicht 14 weist ein Textil 15 mit daran befestigten elektrisch leitfähigen Fäden 18 auf. Einer der elektrisch leitfähigen Fäden 18 ist mäanderförmig entlang einer Längserstreckung R₁ der proximalen Schicht angeordnet und eine andere der elektrisch leitfähigen Fäden 18 ist mäanderförmig entlang einer Querstreckung R₂, die quer zur Längserstreckung R₁ verläuft, der proximalen Schicht angeordnet. Es versteht sich, dass die proximale Schicht weniger oder mehr Kreuzungspunkte aufweisen kann und/oder einen anderen Verlauf aufweisen kann.

Die elektrisch leitfähige Fäden 18 kreuzen sich mehrfach und bilden dadurch eine Vielzahl von Kreuzungsstellen 19.

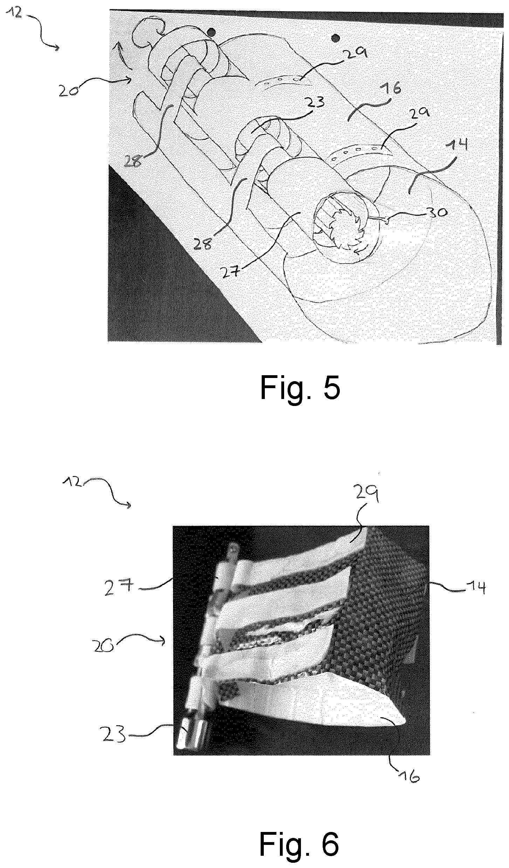

Figur 5 und 6 zeigt eine schematische perspektivische Ansicht einer Messmanschette 12 mit einer proximalen Schicht 14 und einer distalen Schicht 16. Figur 5 zeigt keine Details der proximalen Schicht 14. Jedoch kann diese entsprechend den in den Figuren 1 bis 4 gezeigten Ausführungsbeispielen ausgeführt sein.

In Figur 5 ist die distale Schicht 16 in Form eines Geflechts aus hochfesten Fasern, beispielsweise Aramidfasern, gezeigt.

Figuren 5 und 6 zeigen ferner eine Spannvorrichtung 20 umfassend einen Wickeldorn 23. Die Messmanschette 12 weist Laschen 28 auf, die um einen Bereich des Wickeldorns 23 gewickelt werden können. Die Laschen 28 können Teil der proximalen Schicht 14 und/oder der distalen Schicht 16 sein. Ferner können die Laschen 28 zusätzliche Elemente sein, die mit der proximalen Schicht 14 und/oder mit der distalen Schicht 16 verbunden sind. Der Wickeldorn 23 ist in einer Hülse 27, die über Befestigungselemente 29 mit der distalen Schicht 16 verbunden ist, rotierbar angeordnet.

Die Spannvorrichtung 20 weist ferner einen Arretierstift 30 zum drehfesten Arretieren des Wickeldorns 23.

Durch Rotation der Wickeldorns 23 können die Laschen 28 auf den Wickeldorn 23 aufgewickelt werden, um so die Messmanschette 12 an der Extremität des Patienten zu spannen. Durch Lösen des Arretierstifts 30 kann die Spannung der Messmanschette 12 gelöst oder zumindest reduziert werden.

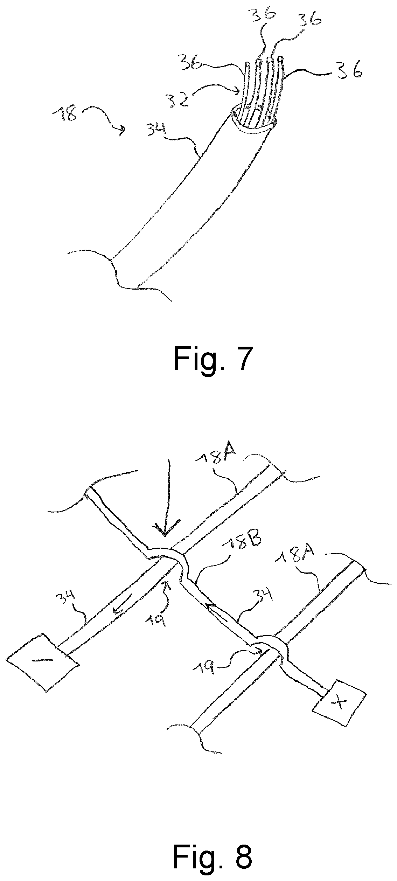

Figur 7 zeigt einen elektrisch leitfähigen Faden 18, der einen leitfähigen Kern 32 und eine den leitfähigen Kern 32 einhüllende Umhüllung 34 aufweist. Der leitfähige Kern 32 umfasst mehrere elektrische Leiter 36.

Figur 8 zeigt im Wesentlichen ein Messprinzip eines erfindungsgemäßen Blutdruckmessgeräts 10. Dabei zeigt Figur 8 Segmente von zwei elektrisch leitfähigen Fäden 18A und 18B, die sich kreuzend angeordnet sind. Dadurch bilden die elektrisch leitfähigen Fäden 18A, 18B eine Vielzahl von Kreuzungspunkten 19, von denen in Figur 8 lediglich zwei abgebildet sind. Die beiden elektrisch leitfähigen Fäden 18A, 18B sind dabei im Wesentlichen senkrecht zueinander angeordnet. Beide elektrisch leitfähige Fäden 18A, 18B umfassen eine Umhüllung 34, die einen elektrisch leitfähigen Kern (nicht abgebildet) einhüllen. Denkbar ist jedoch auch, nur einen der beiden elektrisch leitfähige Fäden 18A, 18B mit einer Umhüllung 34 zu versehen.

Der elektrisch leitfähige Faden 18A ist mit einem Minuspol (-), bzw. einer Masse, und der elektrisch leitfähige Faden 18B ist mit einem Pluspol (+) einer Spannungsquelle (nicht dargestellt) elektrisch verbunden.

Durch Druckeinwirkung auf einen der beiden Kreuzungsstellen 19 wird die Umhüllung 34 zumindest einer der beiden elektrisch leitfähigen Fäden 18A, 18B an der Kreuzungsstelle 19 komprimiert, wodurch sich die elektrisch leitfähigen Kerne der elektrisch leitfähigen Fäden 18A, 18B zumindest an der Kreuzungsstelle 19 nähern. Dadurch sinkt der elektrische Widerstand zwischen den beiden elektrisch leitfähigen Fäden 18A, 18B. Diese Reduktion des elektrischen Widerstands zwischen den beiden elektrisch leitfähigen Fäden 18A, 18B kann gemessen werden, beispielsweise mittels einer Strommessung.

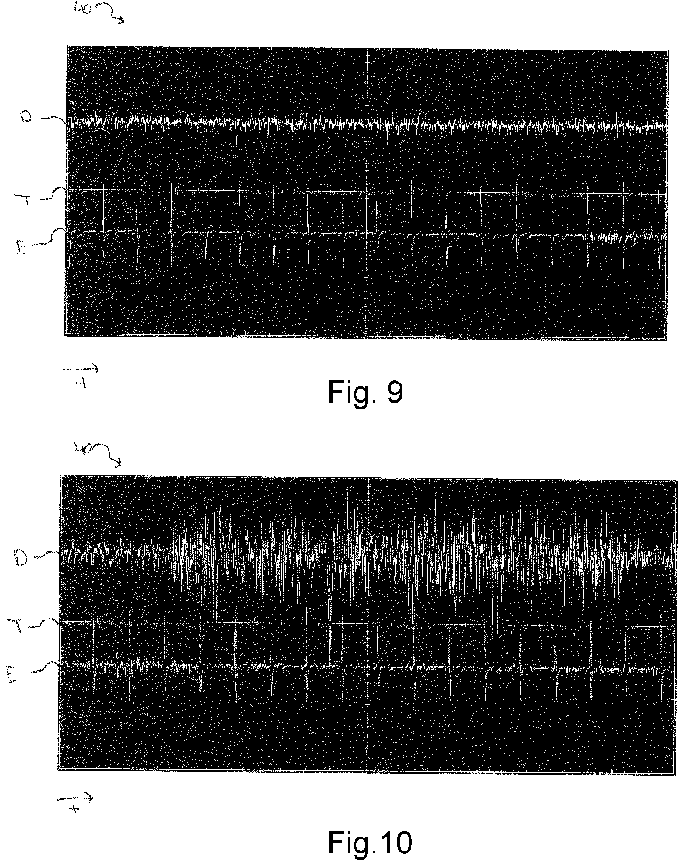

Figur 9 zeigt ein Messdiagramm 40 mit einem mittels eines erfindungsgemäßen Blutdruckmessgeräts 10 erzeugten Drucksignal D über eine Messzeit t.

Ferner umfasst das Messdiagramm 40 ein mittels einer Messelektrode 24 erzeugtes Messsignal T, welches die Muskelaktivitäten in einer Extremität des Patienten erfasst. Das Messsignal T kann beispielsweise mittels elektrischer Myographie (EMG), mit Hilfe beispielsweise einer Messelektrode 24, erzeugt werden.

Außerdem zeigt das Messdiagramm 40 ein Messsignal E eines Elektrokardiogramms (EKG), das beispielsweist mittels eines Messinstruments 24 erfasst wird.

Die Messsignale in dem Messdiagramm 40 in Figur 8 entsprechen einem Ruhezustand des Patienten.

Figur 10 zeigt das Messdiagramm 40 aus Figur 9. Im Gegensatz zu den Messsignalen D, T und E aus Figur 8, entsprechen die Messsignale D, T und E in Figur 9 einem leicht angespannten Zustand der Extremität 26, an der das Blutdruckmessgerät 10 angelegt ist.

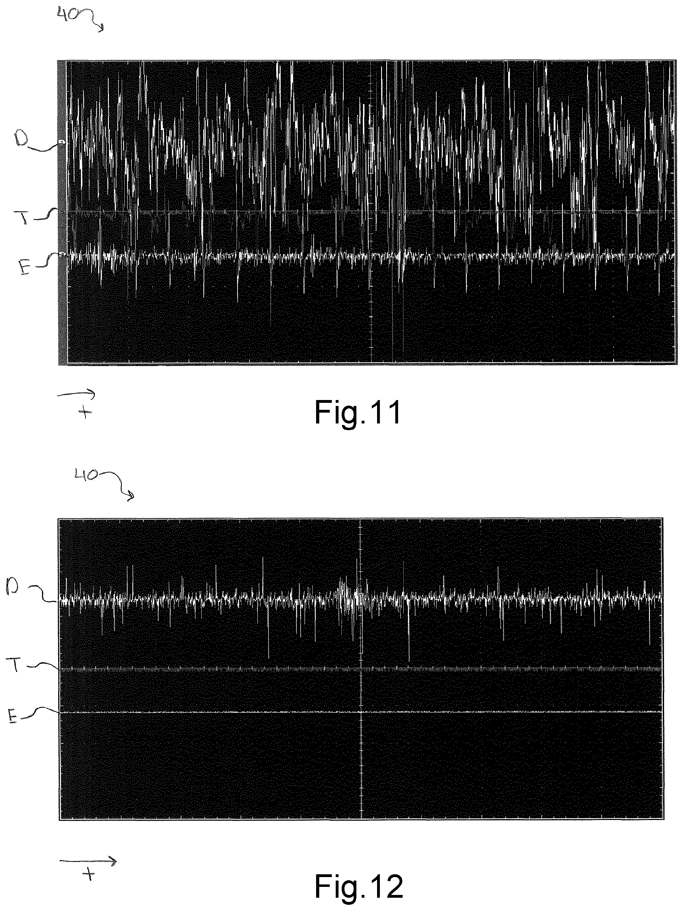

Figur 11 zeigt das Messdiagramm 40 in einem stark angespannten Zustand der Extremität, an der das Blutdruckmessgerät 10 angelegt ist.

Figur 12 zeigt hingegen die Messsignale D, T und E in einem Zustand, in dem die Messmanschette 12 nicht an einer Extremität 26 des Patienten angelegt ist. Stattdessen wurde auf die proximale Schicht 14 der Messmanschette 12 durch einen Menschen exhaliert bzw. auf diese wurde ,gepustet'. Das Drucksignal D umfasst somit lediglich die Detektion der durch die Exhalation bewirkte Druckeinwirkung auf die proximale Schicht.

Mittels der zusätzlichen Messsignale T und E können die Einflüsse der Muskelaktivitäten und der Herzfrequenz auf die Druckeinwirkung auf die proximale Schicht 14 isoliert werden und aus dem Drucksignal D korrigiert werden.

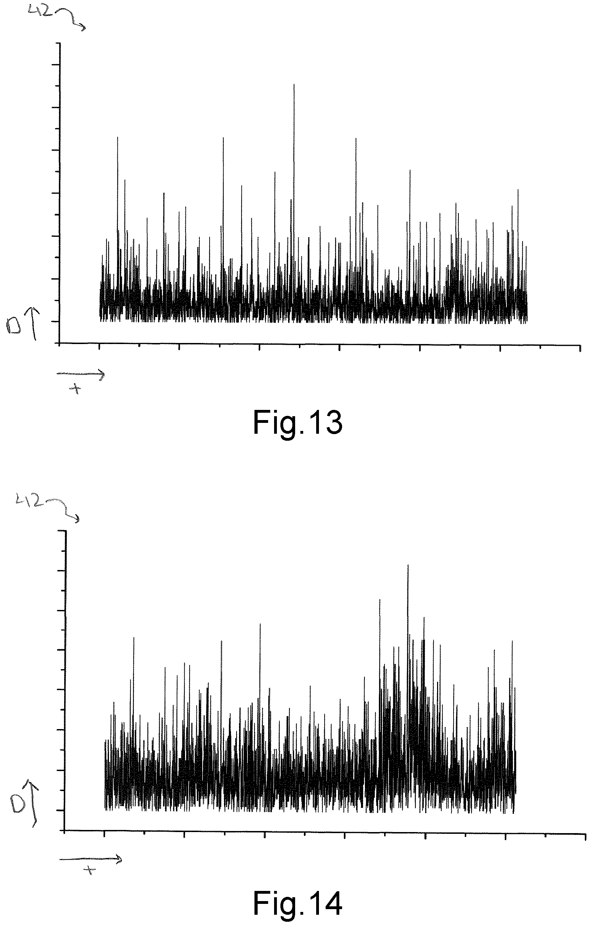

Figuren 13 und 14 zeigen jeweils ein Messdiagramm 42 mit einem derartigen korrigierten Drucksignal D.

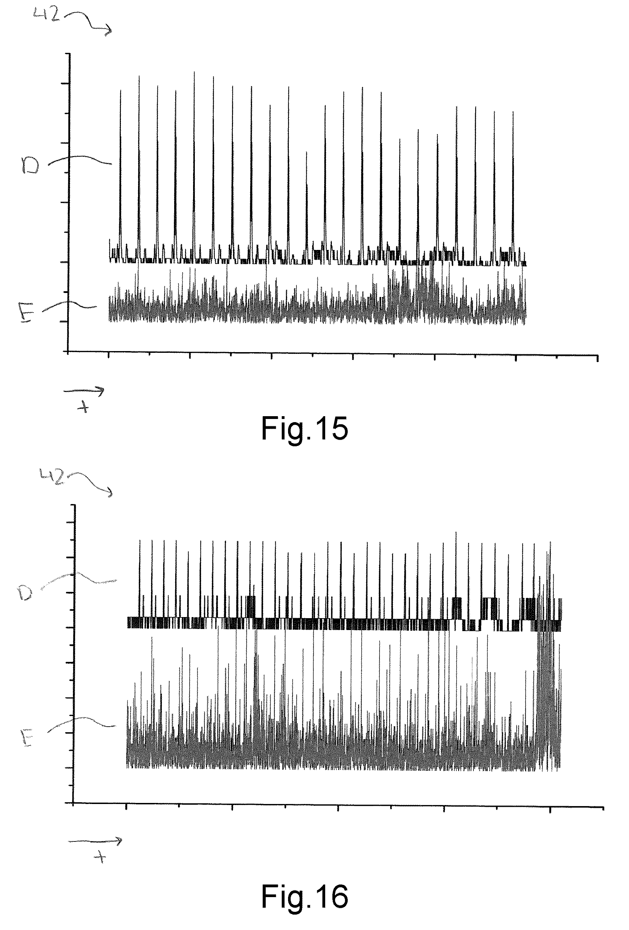

Figuren 15 und 16 zeigen ein korrigiertes Drucksignal D, welches anhand eines ebenfalls in Figuren 14 und 15 gezeigten Messsignals E eines Elektrokardiogramms (EKG) korrigiert wurde.

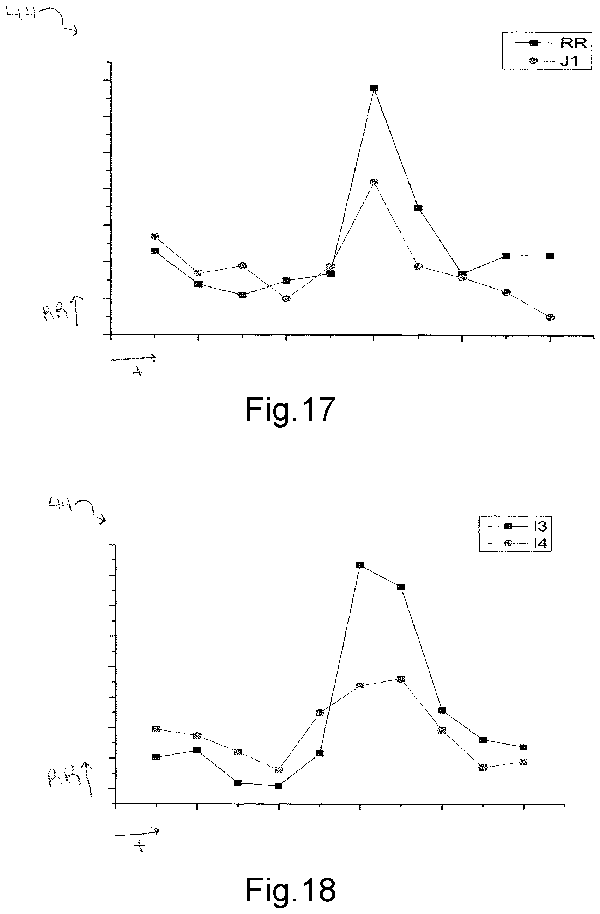

Figur 17 zeigt ein Messdiagramm 44, das zwei Messkurven, die einen mittels eines (eingangs beschriebenen) konventionellen Blutdruckmessgeräts bestimmten Blutdruckverlauf RR über die Zeit t darstellen, umfasst. Die in Figur 17 gezeigten Messkurven dienen als Referenz für die Blutdruckmessung mittels des hierin offenbarten Blutdruckmessgeräts 10. Figur 18 hingegen zeigt ein Messdiagramm 44, das zwei Messkurven eines mittels des hierin offenbarten Blutdruckmessgeräts 10 bestimmten Blutdruckverlaufs RR über die Zeit t darstellt. Der jeweilige Blutdruckverlauf RR kann systolische oder diastolische Blutdruckwerte aufweisen. Die Kurven zeigen jeweils einen sprunghaften Anstieg der Blutdrucks RR ca. in der Mitte des Diagramms. Dieser sprunghafte Anstieg des Blutdrucks RR wurde durch das Durchführen einer sportlichen Betätigung hervorgerufen.

### Bezugszeichenliste

- 10: Blutdruckmessgerät
- 12: Messmanschette
- 14: proximale Schicht
- 15: Textil
- 16: distale Schicht
- 18: elektrisch leitfähiger Faden
- 18A: elektrisch leitfähiger Faden
- 18B: elektrisch leitfähiger Faden
- 19: Kreuzungsstelle
- 20: Spannvorrichtung
- 22: Auswertevorrichtung
- 23: Wickeldorn
- 24: Messinstrument
- 26: Extremität
- 27: Hülse
- 28: Laschen
- 29: Befestigungselemente
- 30: Arretierstift
- 32: leitfähiger Kern
- 34: Umhüllung
- 36: elektrische Leitelemente
- 40: Messdiagramm
- 42: Messdiagramm
- 44: Messdiagramm
- D: Drucksignal
- T: Messsignal
- E: Messsignal
- R₁: Längserstreckung
- R₂: Quererstreckung
- RR: Blutdruck
- L: Länge der Messmanschette

## Patentansprüche

1. Blutdruckmessgerät (10) zum nicht-invasiven Blutdruckmessen, umfassend:
- eine Messmanschette (12), die dazu eingerichtet ist, wenigstens bereichsweise um einen Umfang einer Extremität (26) eines Patienten angelegt zu werden, wobei die Messmanschette (12) wenigstens eine proximale Schicht (14) und eine distale Schicht (16) aufweist, wobei die proximale Schicht (14) zumindest ein druckempfindliches Element (18) aufweist, das dazu eingerichtet ist, unter Druckeinfluss ein diesem Druckeinfluss äquivalentes, kontinuierliches Drucksignal (D) zu erzeugen und wobei die distale Schicht (16) zumindest in Umfangsrichtung unter Druckeinfluss im Wesentlichen nicht dehnbar ist, und
- eine Auswertevorrichtung (22), die dazu eingerichtet ist, in Abhängigkeit von dem Drucksignal (D) den Blutdruck (RR) des Patienten zu bestimmen,
wobei:
das druckempfindliche Element (18) mindestens zwei elektrisch leitfähige Fäden (18) aufweist, deren elektrischer Widerstand durch den Druckeinfluss veränderbar ist, oder die proximale Schicht (14) mindestens eine elektrisch leitfähige Folie und mindestens einen elektrisch leitfähigen Faden (18) aufweist, deren elektrischer Widerstand durch den Druckeinfluss veränderbar ist.

2. Blutdruckmessgerät (10) nach Anspruch 1, wobei die elektrisch leitfähigen Fäden (18) zumindest bereichsweise sich kreuzend angeordnet sind und mindestens eine Kreuzungsstelle (19) bilden, an der sich mindestens zwei elektrisch leitfähige Fäden (18) kreuzend überlagern.

3. Blutdruckmessgerät (10) nach Anspruch 1 oder 2, wobei die elektrisch leitfähigen Fäden (18) einen elektrisch leitfähigen Kern (32) aufweisen und wobei mindestens einer der elektrisch leitfähigen Fäden (18) eine Umhüllung (34) aufweist, die den elektrisch leitfähigen Kern (32) umhüllt, wobei die elektrisch leitfähigen Kerne (32) und die mindestens eine Umhüllung (34) zusammen einen Stromkreis bilden.

4. Blutdruckmessgerät (10) nach einem der vorhergehenden Ansprüche, wobei die distale Schicht (16) aus einem Faserverbundwerkstoff gebildet ist, der hochfeste Fasern, vorzugsweise Aramidfasern, umfasst.

5. Blutdruckmessgerät (10) nach einem der vorhergehenden Ansprüche, wobei die Messmanschette (12) eine Spannvorrichtung (20) zum Spannen der Messmanschette (12) wenigstens bereichsweise um den Umfang der Extremität (26) des Patienten aufweist.

6. Blutdruckmessgerät (10) nach Anspruch 5, wobei die Spannvorrichtung (20) dazu eingerichtet ist, einen Anlegedruck an der Extremität (26) des Patienten zu begrenzen.

7. Blutdruckmessgerät (10) nach Anspruch 6, wobei die Spannvorrichtung (20) dazu eingerichtet ist, den Anlegedruck in Abhängigkeit von dem erzeugten Drucksignal (D) zu begrenzen, wobei der Anlegedruck vorzugsweise auf einen Druckwert, der unterhalb des systolischen Blutdrucks liegt, bevorzugt unterhalb des diastolischen Blutdrucks, begrenzt wird.

8. Blutdruckmessgerät (10) nach einem der vorhergehenden Ansprüche, zusätzlich umfassend mindestens eine Messelektrode (24), die dazu eingerichtet ist, an dem Patienten angelegt zu werden und Muskelaktivitäten, vorzugsweise von einer Vielzahl von Muskeln, des Patienten zu erfassen, vorzugsweise mittels elektrischer Myographie, und an die Auswertevorrichtung (22) bereitzustellen.

9. Blutdruckmessgerät (10) nach einem der vorhergehenden Ansprüche, zusätzlich umfassend mindestens eine Messelektrode (24), die dazu eingerichtet ist, an dem Patienten angelegt zu werden und Atmungszustände des Patienten zu erfassen und an die Auswertevorrichtung (22) bereitzustellen.

10. Blutdruckmessgerät (10) nach einem der vorhergehenden Ansprüche, zusätzlich umfassend eine Herzfrequenz-Messvorrichtung (24), die dazu eingerichtet ist, an dem Patienten angelegt zu werden und die Herzfrequenz des Patienten zu erfassen und ein Signal (E) an die Auswertevorrichtung (22) bereitzustellen.

11. Blutdruckmessgerät (10) nach einem der vorhergehenden Ansprüche, zusätzlich umfassend eine akustische Detektionsvorrichtung (24), die dazu eingerichtet ist, an dem Patienten angelegt zu werden und Geräusche, die durch Muskelkontraktion des Patienten verursacht werden, zu detektieren, vorzugsweise mittels akustischer Myographie, und ein Signal (T) an die Auswertevorrichtung (22) bereitzustellen, wobei die Auswertevorrichtung (22) dazu eingerichtet ist, den Blutdruck (RR) des Patienten zusätzlich in Abhängigkeit der detektierten Geräusche zu bestimmen.

12. Blutdruckmessgerät (10) nach einem der vorhergehenden Ansprüche, zusätzlich umfassend eine Bewegungsmessvorrichtung (24), die dazu eingerichtet ist, Bewegungen des Patienten zu detektieren und ein Signal an die Auswertevorrichtung (22) bereitzustellen, wobei die Auswertevorrichtung (22) dazu eingerichtet ist, den Blutdruck (RR) des Patienten zusätzlich in Abhängigkeit der detektierten Bewegungen des Patienten zu bestimmen.

13. Verfahren zum kontinuierlichen, nicht-invasiven Bestimmen eines Blutdrucks (RR) eines Patienten mit den Schritten:
- Anlegen einer Messmanschette (12), vorzugsweise einer Messmanschette (12) nach einem der Ansprüche 1 bis 12, wenigstens bereichsweise um einen Umfang einer Extremität (26) eines Patienten, wobei die Messmanschette (12) wenigstens eine proximale Schicht (14) und eine distale Schicht (16) aufweist, wobei die proximale Schicht (14) ein druckempfindliches Element (18) aufweist, wobei die distale Schicht (16) zumindest in Umfangsrichtung unter Druckeinfluss im Wesentlichen nicht dehnbar ist, und wobei:
das druckempfindliche Element (18) mindestens zwei elektrisch leitfähige Fäden (18) aufweist, deren elektrischer Widerstand durch den Druckeinfluss veränderbar ist, oder
die proximale Schicht (14) mindestens eine elektrisch leitfähige Folie und mindestens einen elektrisch leitfähigen Faden (18) aufweist, deren elektrischer Widerstand durch den Druckeinfluss veränderbar ist;
- Erzeugen eines Drucksignals durch Druckeinfluss auf das druckempfindliche Element (18), wobei das Drucksignal zu dem Druckeinfluss äquivalent und kontinuierlich ist;
- Bestimmen des Blutdrucks (RR) des Patienten mittels einer Auswertevorrichtung (22) in Abhängigkeit von einem Drucksignal.

## Claims

1. A blood pressure monitor (10) for non-invasive blood pressure measuring, comprising:
- a measuring cuff (12) configured to be applied at least partially around a circumference of an extremity (26) of a patient, wherein the measuring cuff (12) comprises at least one proximal layer (14) and a distal layer (16), wherein the proximal layer (14) comprises at least one pressure-sensitive element (18) configured to generate, under the application of pressure, a continuous pressure signal (D) equivalent to said application of pressure, and wherein the distal layer (16) is substantially non-stretchable under the application of pressure at least in the circumferential direction,
and
- an evaluation device (22) configured to determine the blood pressure (RR) of the patient dependent from the pressure signal (D)
wherein:
the pressure-sensitive element (18) comprises at least two electrically conductive threads (18), the electrical resistance of which is changeable by the application of pressure, or
the proximal layer (14) comprises at least one electrically conductive film and at least one electrically conductive thread (18), the electrical resistance of which is changeable by the application of pressure.

2. The blood pressure monitor (10) according to claim 1, wherein the electrically conductive threads (18) are arranged to cross one another at least partially and form at least one crossing point (19) at which at least two electrically conductive threads (18) cross to overlap one another.

3. The blood pressure monitor (10) according to claim 1 or 2, wherein the electrically conductive threads (18) comprise an electrically conductive core (32) and wherein at least one of the electrically conductive threads (18) comprises a sheath (34) which encloses the electrically conductive core (32), wherein the electrically conductive cores (32) and the at least one sheath (34) together form an electrical circuit.

4. The blood pressure monitor (10) according to any one of the preceding claims, wherein the distal layer (16) is formed from a fiber composite material comprising high-strength fibers, preferably aramid fibers.

5. The blood pressure monitor (10) according to any one of the preceding claims, wherein the measuring cuff (12) comprises a tensioning device (20) for tensioning the measuring cuff (12) at least partially around the circumference of the extremity (26) of the patient.

6. The blood pressure monitor (10) according to claim 5, wherein the tensioning device (20) is configured to limit an application pressure on the extremity (26) of the patient.

7. The blood pressure monitor (10) according to claim 6, wherein the tensioning device (20) is configured to limit the application pressure dependent from the generated pressure signal (D), wherein the application pressure is preferably limited to a pressure value which is below the systolic blood pressure, preferably below the diastolic blood pressure.

8. The blood pressure monitor (10) according to any one of the preceding claims, additionally comprising at least one measuring electrode (24) configured to be applied to the patient and to record muscle activities, preferably of a plurality of muscles, of the patient, preferably by means of electrical myography, and to provide them to the evaluation device (22).

9. The blood pressure monitor (10) according to any one of the preceding claims, additionally comprising at least one measuring electrode (24) configured to be applied to the patient and to detect breathing conditions of the patient and to provide them to the evaluation device (22).

10. The blood pressure monitor (10) according to any one of the preceding claims, additionally comprising a heart rate measuring device (24) configured to be applied to the patient and to detect the heart rate of the patient and to provide a signal (E) to the evaluation device (22).

11. The blood pressure monitor (10) according to any one of the preceding claims, additionally comprising an acoustic detection device (24) configured to be applied to the patient and to detect noises caused by muscle contraction of the patient, preferably by means of acoustic myography, and to provide a signal (T) to the evaluation device (22), wherein the evaluation device (22) is configured to determine the blood pressure (RR) of the patient additionally dependent from the detected noises.

12. The blood pressure monitor (10) according to any one of the preceding claims, additionally comprising a movement measuring device (24) configured to detect movements of the patient and to provide a signal to the evaluation device (22), wherein the evaluation device (22) is configured to determine the blood pressure (RR) of the patient additionally dependent from the detected movements of the patient.

13. A method for the continuous, non-invasive determination of a blood pressure (RR) of a patient, comprising the steps:
- applying a measuring cuff (12), preferably a measuring cuff (12) according to any one of claims 1 to 12, at least partially around a circumference of an extremity (26) of a patient, wherein the measuring cuff (12) comprises at least a proximal layer (14) and a distal layer (16), wherein the proximal layer (14) comprises a pressure-sensitive element (18), wherein the distal layer (16) is substantially non-stretchable under the application of pressure at least in the circumferential direction, and wherein:
the pressure-sensitive element (18) comprises at least two electrically conductive threads (18) the electrical resistance of which can be changed by the application of pressure, or
the proximal layer (14) comprises at least one electrically conductive film and at least one electrically conductive thread (18), the electrical resistance of which can be changed by the application of pressure;
- generating a pressure signal by applying pressure to the pressure-sensitive element (18), the pressure signal being equivalent to the application of pressure and being continuous;
- determining the blood pressure (RR) of the patient by means of an evaluation device (22) dependent from a pressure signal.

## Revendications

1. Tensiomètre (10) pour la mesure non invasive de la pression artérielle, comprenant :
- un brassard de mesure (12) qui est conçu pour être placé au moins partiellement autour de la circonférence d'un membre (26) d'un patient, le brassard de mesure (12) comportant au moins une couche proximale (14) et une couche distale (16), la couche proximale (14) comportant au moins un élément sensible à la pression (18) qui est conçu pour générer, sous l'influence d'une pression, un signal de pression (D) continu équivalent à cette influence de pression, et la couche distale (16) étant sensiblement inextensible sous l'influence d'une pression, au moins dans la direction circonférentielle, et
- un dispositif d'évaluation (22) qui est conçu pour déterminer la pression artérielle (RR) du patient en fonction du signal de pression (D),
dans lequel :
l'élément sensible à la pression (18) comporte au moins deux fils électriquement conducteurs (18) dont la résistance électrique est modifiable sous l'influence de la pression, ou la couche proximale (14) comporte au moins une feuille électriquement conductrice et au moins un fil électriquement conducteur (18) dont la résistance électrique peut être modifiée sous l'influence de la pression.

2. Tensiomètre (10) selon la revendication 1, dans lequel les fils électriquement conducteurs (18) sont disposés de manière à se croiser au moins par endroits et forment au moins un point de croisement (19) où au moins deux fils électriquement conducteurs (18) se superposent en se croisant.

3. Tensiomètre (10) selon la revendication 1 ou 2, dans lequel les fils électriquement conducteurs (18) comportent un noyau électriquement conducteur (32) et au moins l'un des fils électriquement conducteurs (18) comportant une enveloppe (34) qui entoure le noyau électriquement conducteur (32), les noyaux électriquement conducteurs (32) et l'au moins une enveloppe (34) formant ensemble un circuit électrique.

4. Tensiomètre (10) selon l'une des revendications précédentes, dans lequel la couche distale (16) est formée d'un matériau composite fibreux qui comprend des fibres hautement résistantes, de préférence des fibres d'aramide.

5. Tensiomètre (10) selon l'une des revendications précédentes, dans lequel le brassard de mesure (12) comporte un dispositif de serrage (20) pour serrer le brassard de mesure (12) au moins par endroits autour de la circonférence du membre (26) du patient.

6. Tensiomètre (10) selon la revendication 5, dans lequel le dispositif de serrage (20) est conçu pour limiter une pression d'application sur le membre (26) du patient.

7. Tensiomètre (10) selon la revendication 6, dans lequel le dispositif de serrage (20) est conçu pour limiter la pression d'application en fonction du signal de pression (D) généré, la pression d'application étant de préférence limitée à une valeur de pression inférieure à la pression artérielle systolique, de préférence inférieure à la pression artérielle diastolique.

8. Tensiomètre (10) selon l'une des revendications précédentes, comprenant en outre au moins une électrode de mesure (24) qui est conçue pour être appliquée sur le patient et pour détecter l'activité musculaire, de préférence d'une pluralité de muscles, du patient, de préférence au moyen d'une myographie électrique, et pour la transmettre au dispositif d'évaluation (22).

9. Tensiomètre (10) selon l'une des revendications précédentes, comprenant en outre au moins une électrode de mesure (24) qui est conçue pour être appliquée sur le patient et pour détecter les états respiratoires du patient et les transmettre au dispositif d'évaluation (22).

10. Tensiomètre (10) selon l'une des revendications précédentes, comprenant en outre un dispositif de mesure (24) de la fréquence cardiaque qui est conçu pour être appliqué sur le patient et pour détecter la fréquence cardiaque du patient et pour fournir un signal (E) au dispositif d'évaluation (22).

11. Tensiomètre (10) selon l'une des revendications précédentes, comprenant en outre un dispositif de détection acoustique (24) qui est conçu pour être appliqué sur le patient et pour détecter des bruits provoqués par la contraction musculaire du patient, de préférence au moyen d'une myographie acoustique, et pour fournir un signal (T)
au dispositif d'évaluation (22), le dispositif d'évaluation (22) étant conçu pour déterminer en outre la pression artérielle (RR) du patient en fonction des bruits détectés.

12. Tensiomètre (10) selon l'une des revendications précédentes, comprenant en outre un dispositif de mesure de mouvement (24) qui est conçu pour détecter les mouvements du patient et pour fournir un signal au dispositif d'évaluation (22), le dispositif d'évaluation (22) étant conçu pour déterminer en outre la pression artérielle (RR) du patient en fonction des mouvements détectés du patient.

13. Procédé pour déterminer en continu et de manière non invasive la pression artérielle (RR) d'un patient, comprenant les étapes suivantes :
- application d'un brassard de mesure (12), de préférence un brassard de mesure (12) selon l'une des revendications 1 à 12, au moins partiellement autour d'une circonférence d'un membre (26) d'un patient, le brassard de mesure (12) comportant au moins une couche proximale (14) et une couche distale (16), la couche proximale (14) comportant un élément sensible à la pression (18), la couche distale (16) étant essentiellement non extensible sous l'influence de la pression, au moins dans la direction circonférentielle, et :
l'élément sensible à la pression (18) comporte au moins deux fils électriquement conducteurs (18) dont la résistance électrique peut être modifiée sous l'influence de la pression, ou
la couche proximale (14) comporte au moins une feuille électriquement conductrice et au moins un fil électriquement conducteur (18) dont la résistance électrique est modifiable sous l'influence de la pression ;
- génération d'un signal de pression par l'influence de la pression sur l'élément sensible à la pression (18), le signal de pression étant équivalent à l'influence de la pression et continu ;
- détermination de la pression artérielle (RR) du patient à l'aide d'un dispositif d'évaluation (22) en fonction d'un signal de pression.
